# EUROPEAN PATENT APPLICATION

(11) **EP 4 549 585 A1**
(43) Date of publication of application: **07.05.2025**
(21) Application number: 23863057.8
(22) Date of filing: 30.08.2023
(51) Int. Cl.: C12Q 1/6876, A61K 39/395, A61K 45/00, A61K 48/00, A61P 25/28, C12N 1/15, C12N 1/19, C12N 1/21, C12N 5/10, C12N 15/11, C12N 15/13, G01N 33/50, G01N 33/53

(54) **KIT FOR DIAGNOSING ALZHEIMER'S DISEASE AND PHARMACEUTICAL COMPOSITION FOR TREATING ALZHEIMER'S DISEASE**

(30) Priority: 08.09.2022 US 202263374908 P
(71) Applicant: Eisai R&D Management Co., Ltd., Tokyo 112-8088 (JP); Keio University, Tokyo, 108-8345 (JP)
(72) Inventor: WATANABE Hirotaka, Tokyo 160-8582 (JP); OKANO Hideyuki, Tokyo 160-8582 (JP); TAKAHASHI Kentaro, Tsukuba-shi, Ibaraki 300-2635 (JP)
(74) Representative: Hoffmann Eitle
(86) International application number: PCT/JP2023/031575
(87) International publication number: WO 2024/053519

(57) **Abstract**

A kit for diagnosing Alzheimer's disease and a pharmaceutical composition for treating Alzheimer's disease are disclosed, in which EDIL3 or a nucleic acid encoding EDIL3 is used as an index or target.

## Description

### Technical Field

The present disclosure relates to a kit for diagnosing Alzheimer's disease and a pharmaceutical composition for treating Alzheimer's disease.

### Background Art

Alzheimer's disease (Alzheimer's dementia) is one of the major dementias, and is a neurodegenerative disease characterized by a decrease in neurons in the brain parenchyma and neuropathological lesions such as senile plaques (Non Patent Literature 1).

As one of genetic risk factors for onset of Alzheimer's disease, an allele of apolipoprotein E (hereinafter, also described as "APOE") is known. There are three major variants of APOE, ε2, ε3, and ε4, which are distinguished by the amino acids of the 112th and 158th residues (hereinafter, referred to as APOE2, APOE3, and APOE4, respectively). APOE2 is a variant in which the 112th residue is cysteine and the 158^{th} residue is cysteine. APOE3 is a variant in which the 112th residue is cysteine and the 158th residue is arginine. APOE4 is a variant in which the 112th residue is arginine and the 158th residue is arginine. It has been reported that, among them, especially, APOE4 is a genetic risk factor for onset of Alzheimer's disease; and that the probability of onset of Alzheimer's disease in a human subject having a heterozygous APOE4 allele was about 3.68 times and the probability of onset of Alzheimer's disease in a human subject having a homozygous APOE4 allele was about 12.50 times, based on a human subject having a homozygous APOE3 allele (Non Patent Literature 2).

Synapse dysfunction is widely considered to be one of the earliest important lesions seen before neurodegeneration occurs in Alzheimer's disease (Non Patent Literatures 3 and 4). Indeed, in human subjects having APOE4 alleles, immature forms of dendritic spines and abnormal brain activity are seen (Non Patent Literatures 5 and 6). Furthermore, experiments using rodent models have reported that human APOE4 adversely affects synaptic function and morphological integrity (Non Patent Literatures 5, and 7 to 10).

### Citation List

### Non Patent Literature

Non Patent Literature 1: D. J. Selkoe and J. Hardy, "The amyloid hypothesis of Alzheimer's disease at 25 years.", EMBO Mol. Med. 8, 595-608 (2016).
Non Patent Literature 2: Yu Yamazaki et al., "Apolipoprotein E and Alzheimer disease: pathobiology and targeting strategies.", Nat. Rev. Neurol. 15, 501-518 (2019).
Non Patent Literature 3: R. D. Terry et al., "Physical basis of cognitive alterations in Alzheimer's disease: synapse loss is the major correlate of cognitive impairment.", Ann. Neurol. 30, 572-80 (1991).
Non Patent Literature 4: S. T. DeKosky and S. W. Scheff, "Synapse loss in frontal cortex biopsies in Alzheimer's disease: correlation with cognitive severity.", Ann. Neurol. 27, 457-64 (1990).
Non Patent Literature 5: Y. Ji et al., "Apolipoprotein E isoform-specific regulation of dendritic spine morphology in apolipoprotein E transgenic mice and Alzheimer's disease patients.", Neuroscience 122, 305-15 (2003).
Non Patent Literature 6: N. Filippini et al., "Distinct patterns of brain activity in young carriers of the APOE-epsilon4 allele.", Proc. Natl. Acad. Sci. U. S. A. 106, 7209-14 (2009).
Non Patent Literature 7: B. L. Trommer et al., "ApoE isoform affects LTP in human targeted replacement mice.", Neuroreport 15, 2655-8 (2004).
Non Patent Literature 8: K. M. Korwek et al., "ApoE isoform-dependent changes in hippocampal synaptic function.", Mol. Neurodegener. 4, 21 (2009).
Non Patent Literature 9: S. B. Dumanis et al., "ApoE4 decreases spine density and dendritic complexity in cortical neurons in vivo.", J. Neurosci. 29, 15317-22 (2009).
Non Patent Literature 10: Y. Tensaouti et al., "ApoE Regulates the Development of Adult Newborn Hippocampal Neurons.", eNeuro5 (4), ENEURO.0155-18.2018, (2018).
Non Patent Literature 11: N. Ichiyanagi et al., "Establishment of In Vitro FUS-Associated Familial Amyotrophic Lateral Sclerosis Model Using Human Induced Pluripotent Stem Cells.", Stem cell reports 6, 496-510 (2016).
Non Patent Literature 12: Alexandra Grubman et al., "A single-cell atlas of entorhinal cortex from individuals with Alzheimer's disease reveals cell-type-specific gene expression regulation", Nature Neuroscience 22(12): 2087-2097 (2019).
Non Patent Literature 13: Tetsuya Akiyama et al., "Aberrant axon branching via Fos-B dysregulation in FUS-ALS motor neurons.", eBioMedicine 45, 362-378 (2019).

### Summary of Invention

### Technical Problem

An object of the present disclosure is to provide a kit for diagnosing Alzheimer's disease and a pharmaceutical composition for treating Alzheimer's disease.

### Solution to Problem

The present inventors have found that human astrocytes (hereinafter, also described as "iPasts") obtained by differentiating induced pluripotent stem cells (iPSCs) having an APOE4 allele highly expresses EDIL3 (also known as EGF Like Repeats and Discoidin I Like Domains Protein 3, DEL-1 or Developmental Endothelial Locus-1) protein, which is one of proteins contained in the extracellular matrix. The present inventors have also found that when primary neurons are cultured under the condition that EDIL3 is present in a cell culture medium, morphological abnormality, which is a phenotype of Alzheimer's disease, is observed.

The present disclosure relates to, for example, the following.
[1] A kit for diagnosing or assisting diagnosis of Alzheimer's disease, the kit comprising:
   a nucleic acid primer or nucleic acid probe configured to detect an mRNA encoding EDIL3;
   a nucleic acid primer or nucleic acid probe configured to detect a cDNA complementary to the mRNA; or
   an anti-EDIL3 antibody or an EDIL3 binding fragment thereof.
[2] The kit according to [1], comprising an instruction for use.
[3] A diagnostic agent or diagnostic aid for Alzheimer's disease, the agent or aid comprising:
   a nucleic acid primer or nucleic acid probe configured to detect an mRNA encoding EDIL3;
   a nucleic acid primer or nucleic acid probe configured to detect a cDNA complementary to the mRNA; or
   an anti-EDIL3 antibody or an EDIL3 binding fragment thereof.
[4] A nucleic acid primer or nucleic acid probe configured to detect an mRNA encoding EDIL3;
   a nucleic acid primer or nucleic acid probe configured to detect a cDNA complementary to the mRNA; or
   an anti-EDIL3 antibody or an EDIL3 binding fragment thereof,
   for use in the diagnosis of Alzheimer's disease or assistance of diagnosis of Alzheimer's disease.
[5] Use of a nucleic acid primer or nucleic acid probe configured to detect an mRNA encoding EDIL3;
   a nucleic acid primer or nucleic acid probe configured to detect a cDNA complementary to the mRNA; or
   an anti-EDIL3 antibody or an EDIL3 binding fragment thereof,
   in the manufacture of a kit for diagnosing or assisting diagnosis of Alzheimer's disease, or a diagnostic agent or diagnostic aid for Alzheimer's disease.
[6] The kit according to [1] or [2], the diagnostic agent or diagnostic aid according to [3]; the nucleic acid primer, nucleic acid probe, antibody, or EDIL3 binding fragment according to [4]; or the use according to [5], wherein the subject to be diagnosed is a subject having APOE4 genotype.
[7] A method for assisting diagnosis of Alzheimer's disease, the method comprising quantifying an mRNA encoding EDIL3, a cDNA complementary to the mRNA, or EDIL3 in a subject.
[8] A method for assisting diagnosis of Alzheimer's disease, the method comprising: detecting the presence or absence of APOE4 genotype of a subject; and quantifying an mRNA encoding EDIL3, a cDNA complementary to the mRNA, or EDIL3 in the subject.
[9] A method for collecting data for diagnosis of Alzheimer's disease, the method comprising quantifying an mRNA encoding EDIL3, a cDNA complementary to the mRNA, or EDIL3 in a subject.
[10] A method for collecting data for diagnosis of Alzheimer's disease, the method comprising: detecting the presence or absence of APOE4 genotype of a subject; and quantifying an mRNA encoding EDIL3, a cDNA complementary to the mRNA, or EDIL3 in the subject.
[11] A method for diagnosing Alzheimer's disease, the method comprising quantifying an mRNA encoding EDIL3, a cDNA complementary to the mRNA, or EDIL3 in a subject.
[12] A method for diagnosing Alzheimer's disease, the method comprising: detecting the presence or absence of APOE4 genotype of a subject; and quantifying an mRNA encoding EDIL3, a cDNA complementary to the mRNA, or EDIL3 in the subject.
[13] A method for assisting diagnosis of Alzheimer's disease, the method comprising quantifying an mRNA encoding EDIL3, a cDNA complementary to the mRNA, or EDIL3 in a biological sample obtained from a subject.
[14] A method for assisting diagnosis of Alzheimer's disease, the method comprising: detecting the presence or absence of APOE4 genotype of a subject; and quantifying an mRNA encoding EDIL3, a cDNA complementary to the mRNA, or EDIL3 in a biological sample obtained from the subject.
[15] A method for collecting data for diagnosis of Alzheimer's disease, the method comprising quantifying an mRNA encoding EDIL3, a cDNA complementary to the mRNA, or EDIL3 in a biological sample obtained from a subject.
[16] A method for collecting data for diagnosis of Alzheimer's disease, the method comprising: detecting the presence or absence of APOE4 genotype of a subject; and quantifying an mRNA encoding EDIL3, a cDNA complementary to the mRNA, or EDIL3 in a biological sample obtained from the subject.
[17] A method for diagnosing Alzheimer's disease, the method comprising quantifying an mRNA encoding EDIL3, a cDNA complementary to the mRNA, or EDIL3 in a biological sample obtained from a subject.
[18] A method for diagnosing Alzheimer's disease, the method comprising: detecting the presence or absence of APOE4 genotype of a subject; and quantifying an mRNA encoding EDIL3, a cDNA complementary to the mRNA, or EDIL3 in a biological sample obtained from the subject.
[19] The method according to any one of [13] to [18], wherein the quantifying EDIL3 comprises contacting an anti-EDIL3 antibody or an EDIL3 binding fragment thereof with the biological sample obtained from the subject.
[20] The method according to any one of [13] to [19], wherein the biological sample is spinal fluid, plasma, serum, whole blood, saliva, sputum, nipple discharge, urine, sweat, tissue, cell which is optionally a glial cell, or pancreatic fluid.
[21] The method according to any one of [7] to [20], wherein a higher quantified amount of EDIL3 compared to a control indicates that the subject has or is likely to have Alzheimer's disease.
[22] A method for treating Alzheimer's disease, comprising administering a pharmaceutical composition or therapeutic agent for treating Alzheimer's disease to a subject determined to have a higher quantified amount of EDIL3 compared to a control in the method according to any one of [7] to [21].
[23] The method according to [22], wherein the pharmaceutical composition or therapeutic agent comprises a therapeutically effective amount of a substance that inhibits EDIL3.
[24] The method according to [23], wherein the substance that inhibits EDIL3 is a substance that suppresses expression of EDIL3, a substance that promotes degradation of EDIL3, or a substance that inhibits the function of EDIL3.
[25] The method according to any one of [7] to [24], wherein the subject is a subject having APOE4 genotype.
[26] A pharmaceutical composition or therapeutic agent for treating Alzheimer's disease, comprising a substance that inhibits EDIL3.
[27] A substance that inhibits EDIL3 for use in the treatment of Alzheimer's disease.
[28] Use of a substance that inhibits EDIL3 in the manufacture of a pharmaceutical composition or therapeutic agent for treating Alzheimer's disease.
[29] A method for treating Alzheimer's disease, comprising administering a substance that inhibits EDIL3 to a patient in need thereof.
[30] The pharmaceutical composition or therapeutic agent according to [26], the substance according to [27], the use according to [28], or the treatment method according to [29], wherein the substance that inhibits EDIL3 is a substance that suppresses expression of EDIL3, a substance that promotes degradation of EDIL3, or a substance that inhibits the function of EDIL3.
[31] The pharmaceutical composition or therapeutic agent according to [26] or [30], the substance according to [27] or [30], the use according to [28] or [30], or the treatment method according to [29] or [30], wherein the substance that inhibits EDIL3 is a nucleic acid that suppresses expression of EDIL3.
[32] The pharmaceutical composition or therapeutic agent according to [26] or [30], the substance according to [27] or [30], the use according to [28] or [30], or the treatment method according to [29] or [30], wherein the substance that inhibits EDIL3 is an anti-EDIL3 antibody or an antigen-binding fragment thereof.
[33] The pharmaceutical composition or therapeutic agent according to [26] or [30], the substance according to [27] or [30], the use according to [28] or [30], or the treatment method according to [29] or [30], wherein the substance that inhibits EDIL3 is a compound against EDIL3.
[34] The pharmaceutical composition or therapeutic agent, substance, use, or treatment method according to [33], wherein the compound against EDIL3 is a small molecule compound.
[35] The pharmaceutical composition or therapeutic agent according to any one of [26] and [30] to [34] or the use according to any one of [28] and [30] to [34], wherein the pharmaceutical composition or therapeutic agent is a pharmaceutical composition or therapeutic agent for treating a human subject having APOE4 genotype.
[36] The substance according to any one of [27] and [30] to [34] or the treatment method according to any one of [29] to [34], wherein the subject to be treated is a human subject having APOE4 genotype.
[37] A method for screening for a substance that inhibits EDIL3, the method comprising:
   culturing a neuron in a medium containing EDIL3 and a test substance; and
   detecting a phenotype of Alzheimer's disease from the neuron.
[38] A method for screening for a substance that inhibits EDIL3, the method comprising:
   introducing a nucleic acid for expressing EDIL3 into a cell or microorganism;
   culturing the cell or microorganism;
   recovering a culture supernatant obtained by culturing the cell or microorganism;
   culturing a neuron in a medium containing the recovered culture supernatant and a test substance; and
   evaluating a phenotype of Alzheimer's disease from the neuron.
[39] A method for screening for a substance that inhibits EDIL3, the method comprising:
   culturing a cell expressing EDIL3 and a neuron in a medium containing a test substance; and
   detecting a phenotype of Alzheimer's disease from the neuron.
[40] The screening method according to any one of [37]to [39], wherein the neuron is a neuron having APOE4 genotype.
[41] The screening method according to any one of [37] to [40], wherein the substance that inhibits EDIL3 is a substance that suppresses expression of EDIL3, a substance that promotes degradation of EDIL3, or a substance that inhibits the function of EDIL3.
[42] The screening method according to any one of [37] to [41], wherein the substance that inhibits EDIL3 is a substance for treating Alzheimer's disease.
[43] The screening method according to any one of [37] to [42], wherein the substance that inhibits EDIL3 is administered to a human subject having APOE4 genotype suffering from Alzheimer's disease.
[44] A method for screening for an active ingredient of a pharmaceutical composition or therapeutic agent for treating Alzheimer's disease, the method comprising:
   culturing a neuron in a medium containing EDIL3 and a test substance; and
   detecting a phenotype of Alzheimer's disease from the neuron.
[45] A method for screening for an active ingredient of a pharmaceutical composition or therapeutic agent for treating Alzheimer's disease, the method comprising:
   introducing a nucleic acid for expressing EDIL3 into a cell or microorganism;
   culturing the cell or microorganism;
   recovering a culture supernatant obtained by culturing the cell or microorganism;
   culturing a neuron in a medium containing the recovered culture supernatant and a test substance; and
   evaluating a phenotype of Alzheimer's disease from the neuron.
[46] A method for screening for an active ingredient of a pharmaceutical composition or therapeutic agent for treating Alzheimer's disease, the method comprising:
   culturing a cell expressing EDIL3 and a neuron in a medium containing a test substance; and
   detecting a phenotype of Alzheimer's disease from the neuron.
[47] The screening method according to any one of [44]to [46], wherein the neuron is a neuron having APOE4 genotype.
[48] The screening method according to any one of [44] to [47], wherein the evaluating the phenotype of Alzheimer's disease from the neuron comprises evaluating a morphology of the neuron.
[49] The screening method according to any one of [44] to [48], wherein the evaluating the phenotype of Alzheimer's disease from the neuron comprises detecting a change in number of spines of the neuron.
[50] The screening method according to any one of [44]to [49], wherein the pharmaceutical composition or therapeutic agent is administered to a human subject having APOE4 genotype.
[51] A method for screening for a substance that inhibits EDIL3, the method comprising:
   culturing a cell expressing EDIL3 in a medium containing a test substance; and
   evaluating expression or secretion of EDIL3.
[52] The screening method according to [51], wherein the evaluating expression or secretion of EDIL3 is quantifying an mRNA encoding EDIL3, a cDNA complementary to the mRNA, or EDIL3.
[53] The screening method according to [51] or [52], wherein the evaluating expression or secretion of EDIL3 is quantifying EDIL3 in the medium.
[54] The screening method according to [52] or [53], wherein the quantification of EDIL3 is performed using an anti-EDIL3 antibody or an EDIL3 binding fragment thereof.
[55] The screening method according to any one of [51] to [54], wherein the substance that inhibits EDIL3 is a substance that suppresses expression of EDIL3, a substance that promotes degradation of EDIL3, or a substance that inhibits the function of EDIL3.
[56] The screening method according to any one of [51] to [55], wherein the substance that inhibits EDIL3 is a substance for treating Alzheimer's disease.
[57] The screening method according to any one of [51] to [56], wherein the substance that inhibits EDIL3 is administered to a human subject having APOE4 genotype suffering from Alzheimer's disease.
[58] The screening method according to any one of [37] to [57], wherein the substance that inhibits EDIL3 is a nucleic acid that suppresses expression of EDIL3, an anti-EDIL3 antibody or an antigen-binding fragment thereof, or a compound against EDIL3.
[59] The screening method according to [58], wherein the compound against EDIL3 is a small molecule compound.

### Advantageous Effects of Invention

According to an embodiment of the present disclosure, it is possible to provide a kit for diagnosing or assisting diagnosis of Alzheimer's disease. According to an embodiment of the present disclosure, it is possible to provide a diagnostic agent or diagnostic aid for Alzheimer's disease. According to an embodiment of the present disclosure, it is possible to provide a method for diagnosing Alzheimer's disease or a method for assisting diagnosis of Alzheimer's disease. According to an embodiment of the present disclosure, it is possible to provide a method for collecting data for diagnosis of Alzheimer's disease.

According to an embodiment of the present disclosure, it is possible to provide a pharmaceutical composition or therapeutic agent for treating Alzheimer's disease. According to an embodiment of the present disclosure, it is possible to provide a method for treating Alzheimer's disease.

According to an embodiment of the present disclosure, it is possible to provide a method for screening for a substance that inhibits EDIL3. According to an embodiment of the present disclosure, it is possible to provide a method for screening for an active ingredient of a pharmaceutical composition or therapeutic agent for treating Alzheimer's disease.

### Brief Description of Drawings

FIG. 1 shows an outline of a method for generating an allele in which a gene encoding APOE4 was knocked-in to an APOE3 allele (Wild-type allele) in Example 1.
FIG. 2 shows results of gel electricity analysis after amplifying genes of five iPSC clones (#86 to #90) into which the generated allele was introduced by PCR in Example 1.
FIG. 3 shows results of sequencing the genes of the five iPSC clones into which the generated allele was introduced in Example 1.
FIG. 4 shows an outline of a method for inducing iPSCs into astrocytes in Example 1.
FIG. 5 shows representative morphologies of an astrocyte (E4/E4 (#87-5)) derived from an iPSC having APOE4/4 genotype and an astrocyte (E3/E3 (201B7)) derived from 201B7 as an iPSC having APOE3/3 genotype in Example 1.
FIG. 6 shows fluorescence images of iPasts when astrocyte markers S100β, GFAP, and CD44 were immunofluorescence stained and nuclei were stained with Hoechst in Example 1.
FIG. 7 shows the ratio of cells positive in the fluorescence images of S100β, GFAP, and CD44 shown in FIG. 6 in Example 1.
FIG. 8 shows results of measuring the expression levels of mRNAs encoding APOE protein (APOE), an astrocyte marker S100β, and β actin (ACTB) in Example 2.
FIG. 9 shows results of quantifying the expression levels of APOE protein and β-actin expressed by each iPast by Western blotting in Example 2.
FIG. 10 shows results of investigating the amount of APOE in a medium in which each iPast was cultured by an ELISA method in Example 2.
FIG. 11 shows results of mass spectrometry analysis of fragments obtained by treating, with trypsin, APOE in an extra-culture fluid of iPasts having APOE3/3 genotype and an extra-culture fluid of iPasts having APOE4/4 genotype in Example 2.
FIG. 12 is a diagram showing an outline of co-culture of iPasts having APOE4/4 genotype or iPasts having APOE3/3 genotype with mouse primary neurons in Example 3.
FIG. 13 shows representative fluorescence images of mouse primary neurons transfected with Actin-GFP in Example 3.
FIG. 14 shows fluorescence images of dendrites of mouse primary neurons co-cultured with iPasts having APOE4/4 genotype or iPasts having APOE3/3 genotype in Example 3.
FIG. 15 shows the number of dendritic spines observed per µm of dendrite in a fluorescence image acquired in a similar manner to FIG. 14 in Example 3.
FIG. 16 shows the number of dendritic spines observed per µm of dendrite in a fluorescence image acquired in a similar manner to FIG. 14 for each dendritic spine shape.
FIG. 17 shows results of clustering analysis of genes having different expression levels between iPasts having APOE4/4 genotype and iPasts having APOE3/3 genotype in Example 4 as a heatmap for the z-score of logFC of the iPasts having APOE4/4 genotype relative to the iPasts having APOE3/3 genotype.
FIG. 18 shows results of principal component analysis (PCA) on the transcriptome of the iPasts having APOE3/3 genotype and the iPasts having APOE4/4 genotype in Example 4.
FIG. 19 is a diagram showing the results of FIG. 17 as a two-dimensional plot in Example 4.
FIG. 20 shows results of quantification by quantitative reverse transcription PCR of CDKN1A and EDIL3 whose expression levels were increased in the iPasts having APOE4/4 genotype, and β actin as an index of the number of cells in Example 4.
FIG. 21 shows results of measuring the expression levels of genes known to be specifically expressed in Pan-Reactive, A1-like reactive (A1 specific), or A2-like reactive (A2 specific) astrocytes in Example 5 by the same method as in Example 4.
FIG. 22 shows results of measuring the expression levels of genes known to be specifically expressed in Pan-Reactive, A1-like reactive (A1 specific), or A2-like reactive (A2 specific) astrocytes by the same method as in Example 4, in Example 5.
FIG. 23 is a diagram showing a Venn diagram showing an overlap of 119 mRNAs found in Example 4 in which the RNA amount was increased in the iPasts having APOE4/4 genotype as compared with that in the iPasts having APOE3/3 genotype, and 491 genes found in the genome-wide association analysis in Non Patent Literature 12 as genes whose single nucleotide polymorphism may be associated with Alzheimer's disease, in Example 6.
FIG. 24 shows results of evaluating the amount of EDIL3 protein in a lysated iPast and a culture medium (CM) in which the iPasts were cultured, by fragment analysis, in Example 6.
FIG. 25 shows results of evaluating the expression level of EDIL3 by Western blotting for the lysated iPast in Example 6.
FIG. 26 shows results of evaluating the amount of EDIL3 secreted into the culture medium in which iPasts were cultured by Western blotting in Example 6.
FIG. 27 shows results of evaluating the amount of EDIL3 in HEK293 cells transduced with EDIL3 and a culture medium thereof by Western blotting in Example 7.
FIG. 28 shows a protocol in which mouse primary neurons cultured in a culture medium in which HEK293 cells transduced with EDIL3 were cultured during the period of Days 16 to 21 of culture (DIV 16 to 21), and the influence of exposure to EDIL3 on the mouse primary neurons was evaluated in Example 7.
FIG. 29 shows fluorescence images of Actin-GFP in dendrites of mouse primary neurons after culture in the culture medium in which HEK293 cells transduced with EDIL3 were cultured, and results of quantifying the number of dendritic spines per µm in Example 7.
FIG. 30 shows results of quantifying the number of dendritic spines per µm, for each dendritic spine shape, in dendrites of mouse primary neurons after culture in the culture medium in which HEK293 cells transduced with EDIL3 were cultured in Example 7.
FIG. 31 is a diagram showing the expression level of EDIL3 in iPasts having APOE4/4 genotype into which shEDIL3 or shcontrol was introduced in Example 8.
FIG. 32 shows fluorescence images of Actin-GFP in dendrites of mouse primary neurons after culture in a culture medium in which iPasts into which shEDIL3 or shcontrol was introduced was cultured, and results of quantifying the number of dendritic spines per µm in Example 8.
FIG. 33 is a diagram showing fluorescence images of immunofluorescence staining of the parietal lobe section of the postmortem brain of Alzheimer's disease patients or humans who did not show a symptom of Alzheimer's disease in Example 9.
FIG. 34 is a diagram showing the area of amyloid plaques in the brain of Alzheimer's disease patients in Example 9.
FIG. 35 is a diagram showing the proportion of GFAP-positive cells according to the distance from amyloid plaques in the brain of the Alzheimer's disease patients in Example 9.
FIG. 36 is a diagram showing the fluorescence intensity derived from a labeled anti-EDIL3 antibody according to the distance from amyloid plaques in the brain of the Alzheimer's disease patients in Example 9.

### Description of Embodiments

Hereinafter, embodiments for carrying out the present disclosure will be described, but the present disclosure is not limited to the following embodiments.

The terms "a," "an," "the," and similar terms used in the context of the present disclosure (especially in the context of the claims) are to be construed to cover both the singular and the plural, unless otherwise indicated herein or clearly contradicted by context.

### <First Aspect>

A first aspect of the present disclosure is a kit for diagnosing or assisting diagnosis of Alzheimer's disease, the kit including: a nucleic acid primer or nucleic acid probe configured to detect an mRNA encoding EDIL3; a nucleic acid primer or nucleic acid probe configured to detect a cDNA complementary to the mRNA; or an anti-EDIL3 antibody or an EDIL3 binding fragment thereof. The first aspect of the present disclosure can also be, in an embodiment, a diagnostic agent or diagnostic aid for Alzheimer's disease, the agent including: a nucleic acid primer or nucleic acid probe configured to detect an mRNA encoding EDIL3; a nucleic acid primer or nucleic acid probe configured to detect a cDNA complementary to the mRNA; or an anti-EDIL3 antibody or an EDIL3 binding fragment thereof.

EDIL3 (EGF Like Repeats And Discoidin Domains 3) is one of proteins constituting the extracellular matrix, also known as DEL1 (Developmental endothelial locus-1). It is known that EDIL3 secreted extracellularly has a role of controlling epithelial-mesenchymal transition (EMT), and the like. EDIL3 is biosynthesized and secreted by a wide range of cells in the human body, and among them, a high amount of mRNA is expressed particularly in cells of the brain, and secreted EDIL3 is known to be distributed also in the blood and the like. Human EDIL3 is known to have two isoforms, isoform 1 represented by SEQ ID NO: 1 and isoform 2 represented by SEQ ID NO: 2. EDIL3 according to the present disclosure is preferably mammalian EDIL3, and more preferably human EDIL3, and the human EDIL3 may be either isoform 1 or 2.

The present inventors have found that neurons exposed to EDIL3 in a culture environment exhibit a phenotype of Alzheimer's disease. More specifically, the present inventors have found that a decrease in density of the number of dendritic spines is seen in neurons exposed to EDIL3 in a culture environment. Therefore, without wishing to be bound by any theory, it can be predicted that a subject has a possibility of suffering from Alzheimer's disease by measuring the content of EDIL3 having the action of causing neuronal morphological abnormalities associated with Alzheimer's disease due to the presence in an environment where neurons are present, or a nucleic acid encoding EDIL3, for example, based on a high content of EDIL3 or a nucleic acid encoding EDIL3 in a sample collected from the subject. That is, the first aspect of the present disclosure can also be, in an embodiment, a method for assisting diagnosis of Alzheimer's disease, a method for collecting data for diagnosis of Alzheimer's disease, or a method for diagnosing Alzheimer's disease, including quantifying an mRNA encoding EDIL3, a cDNA complementary to the mRNA, or EDIL3 in a subject. In these cases, the quantifying EDIL3 may comprise contacting an anti-EDIL3 antibody or an EDIL3 binding fragment thereof with a biological sample obtained from the subject.

The mRNA encoding EDIL3 in the nucleic acid primer or nucleic acid probe that detects the mRNA encoding EDIL3 is not limited as long as it is a nucleic acid that gives EDIL3 by being translated, and may be, for example, an mRNA encoding a protein having an amino acid sequence identity of 80% or more, 85% or more, 90% or more, 95% or more, 97% or more, 98% or more, 99% or more, or 100% with EDIL3 represented by SEQ ID NO: 1 or SEQ ID NO: 2, in another embodiment, may be an mRNA encoding a protein having an amino acid sequence identity of 95% or more with EDIL3 represented by SEQ ID NO: 1 or SEQ ID NO: 2, and in still another embodiment, may be an mRNA encoding EDIL3 represented by SEQ ID NO: 1 or SEQ ID NO: 2. In these cases, EDIL3 encoded by such a nucleic acid may have its physiological function (e.g., binding properties to an integrin). In addition, detecting the mRNA encoding EDIL3 by a nucleic acid primer or nucleic acid probe that detects the mRNA encoding EDIL3 may be either directly or indirectly detecting the mRNA encoding EDIL3 (that is, detecting the mRNA encoding EDIL3 itself), but may be directly detecting in another embodiment. Indirectly detecting the mRNA encoding EDIL3 may be detecting a nucleic acid that is spliced to give the mRNA encoding EDIL3, i.e., an mRNA precursor of the mRNA encoding EDIL3. Examples of the mRNA precursor of the mRNA encoding EDIL3 comprise a nucleic acid shown in SEQ ID NO: 3 or SEQ ID NO: 4. Examples of the mRNA encoding EDIL3 obtained by splicing of the mRNA precursor represented by SEQ ID NO: 3 or SEQ ID NO: 4 comprise a nucleic acid shown in SEQ ID NO: 5 which is an mRNA encoding isoform 1 (SEQ ID NO: 1) of EDIL3 or a nucleic acid shown in SEQ ID NO: 6 which is an mRNA encoding isoform 2 (SEQ ID NO: 2) of EDIL3. Therefore, in an embodiment of the first aspect of the present disclosure, the nucleic acid primer or nucleic acid probe that detects the mRNA encoding EDIL3 may be a nucleic acid primer or nucleic acid probe that detects a nucleic acid having a homology of 80% or more, 85% or more, 90% or more, 95% or more, 97% or more, 98% or more, 99% or more, or 100% or more with the nucleic acid shown in SEQ ID NO: 3, SEQ ID NO: 4, SEQ ID NO: 5, or SEQ ID NO: 6, in another embodiment, may be a nucleic acid primer or nucleic acid probe that detects a nucleic acid having a homology of 90% or more with the nucleic acid shown in SEQ ID NO: 3, SEQ ID NO: 4, SEQ ID NO: 5, or SEQ ID NO: 6, in still another embodiment, may be a nucleic acid primer or nucleic acid probe that detects a nucleic acid having a homology of 90% or more with the nucleic acid shown in SEQ ID NO: 5 or SEQ ID NO: 6, in still another embodiment, may be a nucleic acid primer or a nucleic acid probe that detects the nucleic acid shown in SEQ ID NO: 3, SEQ ID NO: 4, SEQ ID NO: 5, or SEQ ID NO: 6 and in still another embodiment, may be a nucleic acid primer or nucleic acid probe that detects the nucleic acid shown in SEQ ID NO: 5 or SEQ ID NO: 6. In these cases, EDIL3 encoded by such a nucleic acid may have its physiological function (for example, properties to an integrin).

The nucleic acid primer or nucleic acid probe that detects a cDNA complementary to the mRNA according to the first aspect of the present disclosure is a cDNA complementary to the mRNA of interest by the nucleic acid primer or nucleic acid probe that detects the mRNA encoding EDIL3 described above. Examples of such a cDNA comprise a nucleic acid shown in SEQ ID NO: 7, which is a cDNA complementary to SEQ ID NO: 5, and a nucleic acid shown in SEQ ID NO: 8, which is a cDNA complementary to SEQ ID NO: 6. Therefore, in an embodiment of the first aspect of the present disclosure, the nucleic acid primer or nucleic acid probe that detects the cDNA complementary to the mRNA encoding EDIL3 may be a nucleic acid primer or nucleic acid probe that detects a nucleic acid having a homology of 80% or more, 85% or more, 90% or more, 95% or more, 97% or more, 98% or more, 99% or more, or 100% to the nucleic acid shown in SEQ ID NO: 7 or SEQ ID NO: 8, in another embodiment, may be a nucleic acid primer or nucleic acid probe that detects a nucleic acid having a homology of 90% or more to the nucleic acid shown in SEQ ID NO: 7 or SEQ ID NO: 8, and in still another embodiment, may be a nucleic acid primer or nucleic acid probe that detects the nucleic acid shown in SEQ ID NO: 7 or SEQ ID NO: 8. In these cases, EDIL3 encoded by an mRNA complementary to such a cDNA may have its physiological function (e.g., properties to an integrin).

The nucleic acid primer according to the present disclosure may be any nucleic acid primer that can bind to DNA or RNA to promote the replication reaction of nucleic acid by DNA polymerase or RNA polymerase. DNA or RNA to be detected present in a solution is amplified by such a nucleic acid primer, and then the nucleic acid concentration of the solution is measured by absorbance measurement or the like, whereby the DNA or RNA to be detected can be evaluated (detected and/or quantified). The base length of the nucleic acid primer according to the present disclosure is not particularly limited, and may be, for example, 5 bases or more, 8 bases or more, 10 bases or more, 12 bases or more, 13 bases or more, 14 bases or more, 15 bases or more, 16 bases or more, 17 bases or more, or 18 bases or more, and may be 100 bases or less, 50 bases or less, 40 bases or less, 35 bases or less, 30 bases or less, 27 bases or less, 25 bases or less, 24 bases or less, 23 bases or less, or 22 bases or less. Examples of the base length range may be 5 to 100 bases, 8 to 50 bases, 10 to 40 bases, 12 to 35 bases, 13 to 30 bases, 14 to 27 bases, 15 to 25 bases, 16 to 24 bases, 17 to 23 bases, or 18 to 22 bases. Furthermore, the nucleic acid primer according to the first aspect of the present disclosure has a region (complementary region) complementary to the mRNA or cDNA to be detected, and a ratio at which the sequence of the complementary region and the sequence of the nucleic acid primer binding site of the mRNA or cDNA to be detected are complementary may be 80% or more, 85% or more, 90% or more, 95% or more, 97% or more, 98% or more, 99% or more, or 100% of a base sequence (number of bases) belonging to the complementary region, and may be 90% or more in another embodiment, and may be 100% in still another embodiment. The base length of the complementary region in the nucleic acid primer according to the first aspect of the present disclosure is not particularly limited, and may be, for example, 5 to 100 bases, 8 to 50 bases, 10 to 40 bases, 12 to 35 bases, 13 to 30 bases, 14 to 27 bases, 15 to 25 bases, 16 to 24 bases, 17 to 23 bases, or 18 to 22 bases. Furthermore, the nucleic acid primer according to the first aspect of the present disclosure may have, in addition to the complementary region, a region (additional region) that is not complementary to the sequence of the complementary region. The additional region may be provided at the 5' end and/or the 3' end of the primer, and the base length of the additional region may be, for example, 1 base, 2 bases, 3 bases, 4 bases, 5 bases, 6 bases, 7 bases, 8 bases, 9 bases, or 10 bases.

A specific example of the nucleic acid primer according to the first aspect of the present disclosure can comprise a nucleic acid primer having a complementary region to the nucleic acid set forth in at least one of SEQ ID NOs: 3 to 8. The nucleic acid primer according to the first aspect of the present disclosure may be a nucleic acid comprising a base sequence having a homology of 80% or more, 85% or more, 90% or more, 95% or more, 97% or more, 98% or more, 99% or more, or 100% with a nucleic acid shown in SEQ ID NO: 9 or SEQ ID NO: 10 in another embodiment, may be a nucleic acid comprising a base sequence having a homology of 90% or more with the nucleic acid shown in SEQ ID NO: 9 or SEQ ID NO: 10 in still another embodiment, and may be a nucleic acid comprising a base sequence having a homology of 100% with the nucleic acid shown in SEQ ID NO: 9 or SEQ ID NO: 10 or a nucleic acid consisting of a base sequence having a homology of 90% or more with the nucleic acid shown in SEQ ID NO: 9 or SEQ ID NO: 10 in still another embodiment.
SEQ ID NO: 9: AGCATACCGAGGGGATACATT
SEQ ID NO: 10: CAAGGCTCAACTTCGCATTCA

The nucleic acid probe according to the present disclosure is not particularly limited as long as it is capable of binding to DNA or RNA and evaluating (detecting and/or quantifying) the DNA or RNA bound by a signal emitted by the nucleic acid probe, and specific examples thereof comprise a nucleic acid probe that can be used in a fluorescence in situ hybridization (FISH) method of detecting the presence of DNA or RNA by a fluorescence signal or an invader assay method, and a nucleic acid probe that can be used in a rapid amplification of cDNA ends (RACE) method of detecting the presence of a nucleic acid through amplification of a part of DNA or RNA by reverse transcription polymerase chain reaction (RT-PCR). The nucleic acid probe according to an embodiment of the first aspect of the present disclosure has a region (complementary region) complementary to the mRNA or cDNA to be detected, and a ratio at which the sequence of the complementary region and the sequence of the nucleic acid probe binding site of the mRNA or cDNA to be detected are complementary may be 80% or more, 85% or more, 90% or more, 95% or more, 97% or more, 98% or more, 99% or more, or 100% of a base sequence (number of bases) belonging to the complementary region, and may be 90% or more in another embodiment, and may be 100% in still another embodiment. The base length of the complementary region in the nucleic acid probe according to the first aspect of the present disclosure is not particularly limited, and may be, for example, 5 to 100 bases, 8 to 50 bases, 10 to 40 bases, 12 to 35 bases, 13 to 30 bases, 14 to 27 bases, 15 to 25 bases, 16 to 24 bases, 17 to 23 bases, or 18 to 22 bases. A specific example of the nucleic acid probe according to the first aspect of the present disclosure can comprise a nucleic acid probe having a complementary region to the nucleic acid set forth in at least one of SEQ ID NOs: 3 to 8.

The anti-EDIL3 antibody according to the present disclosure is not particularly limited as long as it is an antibody capable of binding to EDIL3. As the anti-EDIL3 antibody, commercially available products can be used, and such an anti-EDIL3 antibody can be purchased from, for example, Abcam (ab190692) and Thermo Fisher Scientific (Invitrogen, PA5-27994).

The EDIL3 binding fragment according to the present disclosure is not particularly limited as long as it is an antigen-binding fragment capable of binding to EDIL3, and may be, for example, an F(ab')² fragment, an Fab' fragment, an Fab fragment, or an Fv fragment.

In an embodiment, the anti-EDIL3 antibody or EDIL3 binding fragment thereof may be labeled to detect a signal. Examples of such labeling comprise labeling with a fluorescent dye and labeling with a protein tag such as a FLAG tag.

According to the first aspect of the present disclosure, quantifying an mRNA encoding EDIL3, a cDNA complementary to the mRNA, or EDIL3 in a subject makes it possible to assist diagnosis of Alzheimer's disease, collect data for diagnosis of Alzheimer's disease, or diagnose Alzheimer's disease, and specifically, to assist diagnosis of, collect data for diagnosis of, or diagnose affection, onset, or progression of Alzheimer's disease. Hereinafter, an embodiment will be described.

The subject according to the present disclosure may be a human subject suffering from Alzheimer's disease (AD, Alzheimer's dementia) or a human subject having a possibility of suffering from Alzheimer's disease. In these cases, whether or not a human subject is suffering from or has a possibility of suffering from Alzheimer's disease may be determined by a method usually performed by those skilled in the art. The human subject suffering from or having a possibility of suffering from Alzheimer's disease may be, for example, a human subject in which a decrease in cognitive function or a possibility thereof has been suggested by a cognitive function test such as a Mini Mental State Examination (MMSE), may be a human subject in which deposition of proteins known to accumulate in Alzheimer's disease patients such as amyloid β and tau protein in the brain or an abnormal concentration of the proteins in spinal fluid is observed, or may be a human subject in which a decrease in cerebral metabolic function or atrophy of brain tissue is observed. The Alzheimer's disease in these cases may be sporadic or familial Alzheimer's disease, and, in another embodiment, may be sporadic Alzheimer's disease. Furthermore, the Alzheimer's disease in these cases may be late-onset Alzheimer's disease (LOAD) that develops at age of 65 years or older or early-onset Alzheimer's disease (EOAD) that develops at age of less than 65 years, and, in another embodiment, may be late-onset Alzheimer's disease. In other words, the subject in an embodiment of the first aspect of the present disclosure may be either 65 years old or over or under 65 years old, and in another embodiment, may be 65 years old or over.

The subject according to the present disclosure may be a subject having APOE4 genotype (APOE4-positive subject), in an embodiment. Apolipoprotein E (hereinafter, also described as "APOE") is a lipid-binding protein having low density lipoprotein receptor binding properties and is one of proteins involved in lipid metabolism. As one of genetic risk factors for onset of Alzheimer's disease, an APOE allele is known. There are three major variants of APOE, ε2, ε3, and ε4, which are distinguished by the amino acids of the 112th and 158th residues (hereinafter, referred to as APOE2, APOE3, and APOE4, respectively). APOE2 is a variant in which the 112th residue is cysteine and the 158th residue is cysteine. APOE3 is a variant in which the 112th residue is cysteine and the 158th residue is arginine. APOE4 is a variant in which the 112th residue is arginine and the 158th residue is arginine. It has been reported that, among them, especially, APOE4 is a genetic risk factor for onset of Alzheimer's disease; and that the probability of onset of Alzheimer's disease in a human subject having a heterozygous APOE4 allele was about 3.68 times and the probability of onset of Alzheimer's disease in a human subject having a homozygous APOE4 allele was about 12.50 times, based on a human subject having a homozygous APOE3 allele (Non Patent Literature 2). The present inventors have found that EDIL3 is particularly abundantly secreted in cells of a subject having APOE4 genotype, and that, when cultured in a culture solution in which EDIL3 is present, neurons exhibit a phenotype of Alzheimer's disease. In the present disclosure, the subject having APOE4 genotype may be a subject having homozygous or heterozygous APOE4 genotype, and in another embodiment, may be a subject having homozygous APOE4 genotype. In other words, in the present disclosure, the subject having APOE4 genotype may be a subject having APOE4/4, APOE3/4, or APOE2/4 genotype, may be a subject having APOE4/4 or APOE3/4 genotype, and may be a subject having APOE4/4 genotype in a preferred embodiment.

The method for assisting diagnosis of Alzheimer's disease, the method for collecting data for diagnosis of Alzheimer's disease, or the method for diagnosing Alzheimer's disease according to an embodiment of the first aspect of the present disclosure may comprise detecting the presence or absence of an APOE4 allele in the subject (evaluating whether the subject has APOE4 genotype). Detecting the presence or absence of an APOE4 allele in the subject can be performed, for example, by collecting a biological sample from the subject and evaluating the presence or absence of an APOE4 allele in the biological sample. The biological sample in this case is not particularly limited as long as the presence or absence of an APOE4 allele can be evaluated, and may be, for example, spinal fluid, plasma, serum, whole blood, saliva, sputum, nipple discharge, urine, sweat, tissue, cell (including a glial cell), or pancreatic fluid, and may be plasma, serum, or whole blood in another embodiment. The biological sample may be obtained from a region of the brain or central nervous system of the subject, including cerebrospinal fluid. In addition, the method for detecting the presence or absence of APOE4 allele in the subject may be a method usually performed by those skilled in the art, and may be, for example, a method using a sequencer such as a next generation sequencer (NGS), an invader assay method, a fluorescence in situ hybridization (FISH) method, an immunostaining method, a restriction fragment length polymorphism (PCR-RFLP) method, a single strand conformation polymorphism (PCR-SSCP) method, a TaqMan method, or a multiplex ligation-dependent probe amplification (MLPA) method.

In the first aspect of the present disclosure, the quantification of an mRNA encoding EDIL3, a cDNA complementary to the mRNA, or EDIL3 in the subject is usually performed by quantifying the mRNA encoding EDIL3, cDNA complementary to the mRNA, or EDIL3 in a biological sample collected from the subject. That is, the first aspect of the present disclosure can also be, in another embodiment, a method for assisting diagnosis of Alzheimer's disease, a method for collecting data for diagnosis of Alzheimer's disease, or a method for diagnosing Alzheimer's disease, including quantifying an mRNA encoding EDIL3, a cDNA complementary to the mRNA, or EDIL3 in a biological sample obtained from the subject. In these cases, the quantifying EDIL3 may comprise contacting an anti-EDIL3 antibody or an EDIL3 binding fragment thereof with a biological sample obtained from the subject. The biological sample to be collected from the subject is not particularly limited, and may be, for example, spinal fluid, plasma, serum, whole blood, saliva, sputum, nipple discharge, urine, sweat, tissue, cell (including a glial cell), or pancreatic fluid, may be spinal fluid, plasma, serum, or whole blood in another embodiment, and may be spinal fluid in still another embodiment. The biological sample may be obtained from a region of the brain or central nervous system of the subject, including cerebrospinal fluid.

The quantification of an mRNA encoding EDIL3, a cDNA complementary to the mRNA or EDIL3 in a biological sample can be performed by a method usually performed by those skilled in the art, and for example, can be performed according to a protocol usually performed by those skilled in the art by a quantitative PCR (qPCR) method or a quantitative reverse transcription PCR (RT-qPCR) method using the nucleic acid primer according to an embodiment of the first aspect of the present disclosure, a FISH method, an invader assay method or a RACE method using the nucleic acid probe according to an embodiment of the first aspect of the present disclosure, or an ELISA method or a Western blotting method using the anti-EDIL3 antibody or EDIL3 binding fragment thereof according to an embodiment of the first aspect of the present disclosure.

In the first aspect of the present disclosure, in a case where the amount of mRNA encoding EDIL3, cDNA complementary to the mRNA, or EDIL3 in a subject obtained by quantification is high (concentration is high), it is possible to assist diagnosis of, collect data for diagnosis of, or diagnose affection, onset, or progression of Alzheimer's disease in the subject, or possibility or high possibility thereof. More specifically, when the amount of mRNA encoding EDIL3, cDNA complementary to the mRNA, or EDIL3 in a subject is higher than a predetermined value (control), the subject can be subjected to assistance of diagnosis or collection of data for diagnosis that the subject is suffering from or has a possibility of suffering from Alzheimer's disease. The predetermined value may be, for example, the amount of mRNA encoding EDIL3, cDNA complementary to the mRNA, or EDIL3 in a control subject, and the control subject may be a subject not suffering from Alzheimer's disease, an APOE4-negative subject (for example, a subject having APOE3/3 genotype), or an APOE4-negative subject (for example, having APOE3/3 genotype) and not suffering from Alzheimer's disease. In an embodiment, the predetermined value may be an upper limit of 68% or 95% confidence interval when the distribution of the amount of mRNA encoding EDIL3, cDNA complementary to the mRNA, or EDIL3 in the control subject is fitted as normal distribution, and may be a value that is 1.1 times, 1.2 times, 1.3 times, 1.5 times, 1.7 times, 2 times, 3 times, 4 times, 5 times, 7 times, 10 times, 15 times or 20 times the average value or median value of the amount of mRNA encoding EDIL3, cDNA complementary to the mRNA, or EDIL3 in the control subject. In an embodiment, the quantification of an mRNA encoding EDIL3, a cDNA complementary to the mRNA, or EDIL3 in the subject may be performed based on a measured value (for example, absorbance or fluorescence intensity) obtained by the method described above instead of the amount of mRNA encoding EDIL3, cDNA complementary to the mRNA, or EDIL3, or may be performed based on comparison with the predetermined value for the measured value obtained by the same method as described above.

Another aspect of the present disclosure may be a method for treating Alzheimer's disease, the method including administering a pharmaceutical composition or therapeutic agent for treating Alzheimer's disease to a subject determined to have a higher quantified amount of EDIL3 compared to a control in the method for assisting diagnosis of Alzheimer's disease, the method for collecting data for diagnosis of Alzheimer's disease, or the method for diagnosing Alzheimer's disease according to an embodiment of the first aspect of the present disclosure. In an embodiment, the pharmaceutical composition or therapeutic agent in these cases may comprise a substance that inhibits EDIL3 in a therapeutically effective amount according to a second aspect of the present disclosure described later, and the substance that inhibits EDIL3 may be, for example, a substance that suppresses expression of EDIL3, a substance that promotes degradation of EDIL3, or a substance that inhibits the function of EDIL3. In addition, the pharmaceutical composition or therapeutic agent in these cases may comprise, for example, an anti-amyloid β antibody (for example, an anti-amyloid β protofibril antibody) or an agent that inhibits production or accumulation of amyloid β in the brain.

The kit according to the first aspect of the present disclosure may further comprise an instruction for use (package insert), and the instruction for use may be in the form of paper or may be digitized. In an embodiment, the instruction for use may describe contents corresponding to an embodiment of the method for assisting diagnosis of Alzheimer's disease, the method for collecting data for diagnosis of Alzheimer's disease, or the method for diagnosing Alzheimer's disease according to the first aspect of the present disclosure.

### <Second Aspect>

A second aspect of the present disclosure is a pharmaceutical composition or therapeutic agent for treating Alzheimer's disease, comprising a substance that inhibits EDIL3. An embodiment of the second aspect of the present disclosure is the pharmaceutical composition or therapeutic agent for treating Alzheimer's disease, wherein the substance that inhibits EDIL3 is a substance that suppresses expression of EDIL3, a substance that promotes degradation of EDIL3, or a substance that inhibits the function of EDIL3. A particular embodiment of the second aspect of the present disclosure is the pharmaceutical composition or therapeutic agent for treating Alzheimer's disease, which comprises a therapeutically effective amount of a substance that suppresses expression of EDIL3, a substance that promotes degradation of EDIL3, or a substance that inhibits the function of EDIL3.

The "pharmaceutical composition" or "therapeutic agent" in the present disclosure is understood to mean a substance that produces the desired effect in a tissue, animal, mammal, human, or another subject.

In the present disclosure, the "treating" and its derivatives mean therapeutic therapy. With reference to a specific condition, the treating means (1) ameliorating the condition, or one or more biological signs of the condition, (2) interfering with (a) one or more points in a biological cascade leading to or causing the condition, or (b) one or more biological signs of the condition, (3) alleviating one or more symptoms, influences, or side effects associated with the condition, or one or more symptoms, influences, or side effects associated with the condition or treatment thereof, or (4) delaying progression of the condition, or one or more biological signs of the condition. As used herein, the "delaying" progression of Alzheimer's disease means postponing, preventing, decelerating, slowing, stabilizing, and/or prolonging progression of a disease, and/or slowing progression thereof or, if developed once, altering the underlying disease course and/or process. The delay can be of varying length of time, depending on the disease history and/or individual being treated. As will be apparent to those skilled in the art, sufficient or significant delay may encompass prophylaxis in that the individual does not actually develop the disease. A method of "delaying" progression of Alzheimer's disease is a method of reducing the probability of disease onset within a given period of time and/or reducing the extent of a disease within a given period of time, including stabilizing one or more symptoms resulting from the disease (for example, memory, problem resolution, language, computation, visuospatial perception, abnormalities in judgment and/or behavior, inability to care for oneself), as compared with not using the method. Such comparisons may be based on clinical trials using a sufficient number of subjects to generally achieve statistically significant results. The progression of Alzheimer's disease can be detected using standard clinical techniques such as standard neurological examination, patient interview, neuroimaging, detection of changes in specific protein levels in the serum or cerebrospinal fluid (for example, amyloid peptides and/or tau), computed tomography (CT), magnetic resonance imaging (MRI), and/or positron emission tomography (PET) brain imaging of amyloid or tau. The "progression" as used herein may also refer to the progression of a disease that may be initially undetectable, and may include occurrence, recurrence, worsening, and/or onset.

In the present disclosure, the "therapeutically effective amount" means an amount effective to elicit a desired biological or pharmaceutical response in a tissue system, animal, or human. In an embodiment, the therapeutically effective amount means an amount effective, at dosages and for periods of time necessary, to achieve the desired therapeutic result (for example, improvement or prevention of symptoms or prolongation of survival). In an embodiment, the therapeutically effective amount is an amount that does not exceed a maximum tolerated dose.

The "pharmaceutical composition" or "therapeutic agent" of the present disclosure can be administered by injection, oral administration, nasal administration, transdermal administration, pulmonary administration, eye drops, and the like. Examples of the injection comprise an intravenous injection, a subcutaneous injection, an intradermal injection, an intraarterial injection, and an injection such as a local injection into a target cell or organ, and may be, for example, a spinal cord injection. Examples of the dosage form of the pharmaceutical composition or therapeutic agent in the case of oral administration comprise tablets, powders, granules, syrups, capsules, and internal liquid preparations. Examples of the dosage form of the pharmaceutical composition or therapeutic agent in the case of parenteral administration comprise injections, drops, eye drops, ointments, suppositories, suspensions, cataplasms, lotions, aerosols and plasters, and in an embodiment, the dosage form is an injection or a drop. The pharmaceutical composition or therapeutic agent according to the present disclosure can be formulated by, for example, the method described in Japanese Pharmacopoeia 18 (JP), United States Pharmacopoeia (USP), or European Pharmacopoeia (EP).

The present inventors have found that neurons exposed to EDIL3 in the surrounding environment of the cells exhibit a phenotype of Alzheimer's disease. More specifically, the present inventors have found that a decrease in density of the number of dendritic spines is seen in neurons exposed to EDIL3 in the surrounding environment of the cells. Therefore, without wishing to be bound by any theory, it is considered that, if the amount of EDIL3 in the environment in which neurons are present in a subject suffering from Alzheimer's disease can be reduced by a substance that suppresses expression of EDIL3, a substance that promotes degradation of EDIL3, or a substance that inhibits the function of EDIL3, Alzheimer's disease can be treated by suppressing neurological dysfunction (for example, neuronal morphological abnormalities) that is a phenotype of Alzheimer's disease. That is, the second aspect of the present disclosure may be, in an embodiment, a method for treating Alzheimer's disease, the method including administering a substance that inhibits EDIL3 to a patient in need thereof, and in another embodiment, may also be a method for treating Alzheimer's disease, the method including administering a substance that suppresses expression of EDIL3, a substance that promotes degradation of EDIL3, or a substance that inhibits the function of EDIL3 to a patient in need thereof.

In the present disclosure, the substance that inhibits EDIL3 means a substance capable of suppressing the action of EDIL3 on neurons in the administered subject, and may be, for example, a substance that suppresses expression of EDIL3, a substance that promotes degradation of EDIL3, or a substance that inhibits the function of EDIL3.

The substance that suppresses expression of EDIL3 is not particularly limited as long as it is a substance capable of suppressing expression of EDIL3 in the body of the subject. In an embodiment, it may be a nucleic acid that suppresses expression of EDIL3. Examples of the nucleic acid that suppresses expression of EDIL3 comprise an antisense nucleic acid (ASO) targeting EDIL3 or a small interfering RNA (siRNA). The base length of the nucleic acid contained in the antisense nucleic acid or siRNA is not particularly limited as long as it is capable of suppressing expression of EDIL3, and may be, for example, 5 bases or more, 8 bases or more, 10 bases or more, 13 bases or more, 15 bases or more, 17 bases or more, 18 bases or more, 19 bases or more, 20 bases or more, or 21 bases or more, and may be 100 bases or less, 70 bases or less, 50 bases or less, 40 bases or less, 35 bases or less, 30 bases or less, 27 bases or less, 25 bases or less, 24 bases or less, or 23 bases or less. Examples of the base length range may be 5 to 100 bases, 8 to 70 bases, 10 to 50 bases, 13 to 40 bases, 15 to 35 bases, 17 to 30 bases, 18 to 27 bases, 19 to 25 bases, 20 to 24 bases, or 21 to 23 bases. In addition, the nucleic acid that suppresses expression of EDIL3 (for example, antisense nucleic acid or siRNA) according to the second aspect of the present disclosure has a region (complementary region) complementary to the mRNA or cDNA encoding EDIL3, and the ratio at which the sequence of the complementary region and the sequence of the binding site of the nucleic acid that suppresses expression of EDIL3 of the mRNA or cDNA encoding EDIL3 are complementary may be 80% or more, 85% or more, 90% or more, 95% or more, 97% or more, 98% or more, 99% or more, or 100% of a base sequence (number of bases) belonging to the complementary region, and may be 90% or more in another embodiment, and may be 100% in still another embodiment. The base length of the complementary region in the nucleic acid that suppresses expression of EDIL3 according to the second aspect of the present disclosure is not particularly limited, and may be, for example, 5 to 100 bases, 8 to 50 bases, 10 to 40 bases, 12 to 35 bases, 13 to 30 bases, 14 to 27 bases, 15 to 25 bases, 16 to 24 bases, 17 to 23 bases, or 18 to 22 bases. Furthermore, the nucleic acid that suppresses expression of EDIL3 according to the second aspect of the present disclosure may have, in addition to the complementary region, a region (additional region) that is not complementary to the sequence of the complementary region. The additional region may be provided at the 5' end and/or the 3' end of the nucleic acid, and the base length of the additional region may be, for example, 1 base, 2 bases, 3 bases, 4 bases, 5 bases, 6 bases, 7 bases, 8 bases, 9 bases, or 10 bases. The mRNA or cDNA encoding EDIL3 targeted by the antisense nucleic acid or siRNA in these cases is similar to that described in the first aspect of the present disclosure.

The substance that promotes degradation of EDIL3 is not particularly limited as long as it is a substance capable of promoting degradation of EDIL3 in the body of the subject. In an embodiment, the substance that promotes degradation of EDIL3 may be an anti-EDIL3 antibody or an antigen-binding fragment thereof. As such an anti-EDIL3 antibody or antigen-binding fragment thereof, the anti-EDIL3 antibody and antigen-binding fragment thereof described in the first aspect of the present disclosure can be used.

In the present disclosure, the inhibiting the function of EDIL3 means inhibiting the dysfunction of neurons caused by EDIL3. In the present disclosure, the substance that inhibits the function of EDIL3 means a substance that inhibits the dysfunction of neurons caused by EDIL3. The substance that inhibits the function of EDIL3 may be, for example, a compound that inhibits the function of EDIL3 (a compound against EDIL3), and the compound against EDIL3 may be a small molecule compound (for example, a compound having a molecular weight of 1000 or less). In addition, the substance that inhibits the function of EDIL3 may be, for example, a small molecule compound, a nucleic acid, a peptide, or an anti-EDIL3 antibody or an antigen-binding fragment thereof that inhibits the dysfunction of neurons caused by EDIL3, and these may have binding properties to EDIL3 or the EDIL3 binding site in neurons or related proteins thereof.

The substance that inhibits EDIL3 according to the second aspect of the present disclosure may be, in an embodiment, a nucleic acid that suppresses expression of EDIL3, an anti-EDIL3 antibody or antigen-binding fragment thereof, or a compound against EDIL3, and in another embodiment, may be a nucleic acid that suppresses expression of EDIL3, or an anti-EDIL3 antibody or antigen-binding fragment thereof.

According to the second aspect of the present disclosure, Alzheimer's disease can be treated by administering a substance that inhibits EDIL3 to a subject (e.g., a patient in need thereof). The subject according to the second aspect of the present disclosure is similar to the subject described in the first aspect of the present disclosure. The pharmaceutical composition or therapeutic agent according to an embodiment of the second aspect of the present disclosure may be for treatment of a subject having APOE4 genotype. The subject to be treated in the method of treatment according to an embodiment of the second aspect of the present disclosure may be a human subject having then APOE4 genotype. Furthermore, the Alzheimer's disease according to the second aspect of the present disclosure is similar to the Alzheimer's disease described in the first aspect of the present disclosure.

As for the dosage of the substance that inhibits EDIL3 in an embodiment of the second aspect of the present disclosure, when the substance is administered to, for example, a human adult male (body weight: 60 kg), the dosage thereof per day may be 0.0001 µg to 10000 mg/day/person in terms of the amount of the active ingredient.

The pharmaceutical composition or therapeutic agent according to an embodiment of the second aspect of the present disclosure may further comprise an additional component in addition to the substance that inhibits EDIL3, and examples of such additional component comprise additives that are commonly used in the technical field of formulation such as excipients, buffers, stabilizers, antioxidants, binders, disintegrants, fillers, emulsifiers, and fluidizing agents.

### <Third Aspect>

A third aspect of the present disclosure is a method for screening for a substance that inhibits EDIL3, the method including culturing a neuron in a medium containing a test substance (that is, contacting the test substance with a neuron, and exposing a neuron to the test substance). According to the screening method according to the third aspect of the present disclosure, a substance that inhibits EDIL3 is sorted from among test substances. The substance that inhibits EDIL3 sorted by the screening method according to the third aspect of the present disclosure may be a substance that suppresses expression of EDIL3, a substance that promotes degradation of EDIL3, or a substance that inhibits the function of EDIL3. The substance that inhibits EDIL3 sorted by the screening method according to the third aspect of the present disclosure may be, in an embodiment, a nucleic acid that suppresses expression of EDIL3, an anti-EDIL3 antibody or antigen-binding fragment thereof, or a compound against EDIL3. The compound against EDIL3 may be a small molecule compound. Furthermore, the neuron in the screening method according to the third aspect of the present disclosure may be a neuron having APOE4 genotype.

The present inventors have found that when EDIL3 is present in an environment in which neurons are present, a phenotype of Alzheimer's disease appears, which is considered to cause neuron dysfunction. Thus, without wishing to be bound by any theory, the substance that inhibits EDIL3 may exert a therapeutic effect on Alzheimer's disease by inhibiting EDIL3 in the subject administered with the substance. That is, in the third aspect of the present disclosure, the substance that inhibits EDIL3 may be a substance for treating Alzheimer's disease (substances for use as an active ingredient of the pharmaceutical composition or therapeutic agent for treating Alzheimer's disease), or may be a substance to be administered to a human subject having APOE4 genotype and suffering from Alzheimer's disease. In these cases, the "inhibiting EDIL3" and "substance that inhibits EDIL3" are defined in the same manner as described in the second aspect of the present disclosure.

A first embodiment of the third aspect of the present disclosure is a method for screening for a substance that inhibits EDIL3, the method including: culturing a neuron in a medium containingEDIL3 and a test substance; and detecting a phenotype of Alzheimer's disease from the neuron. In addition, the first embodiment of the third aspect of the present disclosure may be a method for screening for an active ingredient of a pharmaceutical composition or therapeutic agent for treating Alzheimer's disease, the method including: culturing a neuron in a medium containing EDIL3 and a test substance; and detecting a phenotype of Alzheimer's disease from the neuron, and the pharmaceutical composition or therapeutic agent may be for administration to a human subject having APOE4 genotype.

In an embodiment, the screening method according to the first embodiment of the third aspect of the present disclosure comprises: culturing a neuron in a medium containing EDIL3 and a test substance (exposure step); and detecting a phenotype of Alzheimer's disease from the neuron (detection step).

In the exposure step, the neuron is exposed to EDIL3 in the presence of the test substance. The neuron is not particularly limited, but is preferably a primary neuron or a neuron derived from iPSC. The primary neuron is preferably a primary neuron derived from a mammal (for example, human, mouse, or rat). The neuron derived from iPSC is preferably a neuron derived from iPSC prepared from a cell of a mammal (for example, human, mouse, or rat). The exposure step can be performed by culturing a neuron in a medium in which EDIL3 and a test substance are added to a medium usually used by those skilled in the art for culturing a neuron. The concentrations of EDIL3 and the test substance in the medium are not particularly limited, and a plurality of media containing EDIL3 and the test substance at different concentrations (e.g. 0.01 µM, 0.1 µM, 1 µM, 10 µM and/or 100 µM) may be tested, and screening may be performed based on the concentration dependence. The exposure time in the exposure step is not particularly limited as long as it is an exposure time at which the phenotype of Alzheimer's disease can be detected at the time of exposure to the same concentration of EDIL3 in the absence of the test substance, and may be, for example, 1 day to 30 days, 2 days to 15 days, or 3 days to 10 days, and the culture conditions may be conditions usually used by those skilled in the art (for example, 37°C, 5%CO₂, and 100% humidity). The number of days of culture (DIV) of the primary neuron used in screening, which is a primary neuron as the neuron, is not particularly limited, and may be, for example, 1 to 50, 3 to 40, 6 to 30, 9 to 27, 11 to 25, 14 to 23, or 16 to 21.

The test substance in the screening method according to the third aspect of the present disclosure is not particularly limited, but may be a substance that has low cell membrane permeability and can continue to be present in the extracellular environment when administered to a human subject because EDIL3 is a protein present in the extracellular matrix. The test substance in the screening method according to the third aspect of the present disclosure may be, for example, a test substance contained in a library composed of (constructed by) a substance expected to have some physiological activity. Examples of such a library comprise a compound library containing a small molecule compound (for example, a molecular weight of 50 or more and 10000 or less or 100 or more and 2000 or less), a nucleic acid library (aptamer library) containing a nucleic acid (for example, 10bp to 100bp or 20bp to 50bp), a peptide library containing a peptide (for example, 4 residues to 50 residues or 6 residues to 20 residues), or an antibody library containing an anti-EDIL3 antibody or an antigen-binding fragment thereof. All of candidate substances contained in such libraries may be used as the test substance, and only some candidate substances selected in advance for cell membrane permeability, in vivo stability, in vivo kinetics, or the like may be used as the test substance.

EDIL3 according to the first embodiment of the third aspect of the present disclosure may be artificially synthesized. In addition, EDIL3 according to the first embodiment of the third aspect of the present disclosure may be secreted from a cell expressing EDIL3. The cell expressing EDIL3 may be a cell expressing EDIL3 under physiological conditions, or may be a cell in which EDIL3 is overexpressed by transduction thereof. However, from the viewpoint of exposing a neuron to a high amount of EDIL3 to easily cause a morphological change, thereby enhancing the screening efficiency, the cell is preferably a cell in which EDIL3 is overexpressed by transduction thereof. That is, the first embodiment of the third aspect of the present disclosure may be a method for screening for a substance that inhibits EDIL3, the method including: introducing a nucleic acid for expressing EDIL3 into a cell or microorganism; culturing the cell or microorganism; recovering a culture supernatant obtained by culturing the cell or microorganism; culturing a neuron in a medium containing the recovered culture supernatant and a test substance; and evaluating a phenotype of Alzheimer's disease from the neuron. In this case, the introducing a nucleic acid for expressing EDIL3 into a cell or microorganism; culturing the cell or microorganism; and recovering a culture supernatant obtained by culturing the cell or microorganism can be performed according to a method usually performed by those skilled in the art using materials and reagents usually used in cell culture and transduction as long as an effective amount of EDIL3 is secreted into the recovered culture supernatant.

In the detection step, the phenotype of Alzheimer's disease is detected and evaluated for the neuron exposed to EDIL3 in the presence of the test substance in the exposure step. As a result of the evaluation in the detection step, a test substance by which the phenotype of Alzheimer's disease in the neuron is not detected or is detected but suppressed is sorted as the substance that inhibits EDIL3 or the active ingredient of the pharmaceutical composition or therapeutic agent for treating Alzheimer's disease.

The evaluating the phenotype of Alzheimer's disease in the neuron in the detection step is not particularly limited as long as it can distinguish whether the phenotype can induce Alzheimer's disease based on the presence or absence or the degree thereof, and may be, for example, evaluating the morphology, transcriptome, metabolite, or proteome of the neuron, or, in another embodiment, may be evaluating the morphology of the neuron from the viewpoint of convenience. When the evaluating the phenotype of Alzheimer's disease in the neuron in the detection step is evaluating the morphology of the neuron, from the viewpoint of convenience of evaluation, the neuron may be fluorescently labeled. The fluorescent labeling may be, for example, transduction of a cytosolically- or membrane-localized fluorescent protein (for example, GFP or DsRed) or introduction of a fluorescent protein into a cytoskeletal protein such as actin. The evaluating the phenotype of Alzheimer's disease in the neuron may comprise, for example, detecting a change in number (density) of spines in the neuron. The present inventors have found that in primary neurons cultured in an environment in which EDIL3 is present, a decrease in density of neuron spines is observed.

As an example of the evaluation in the detection step, for example, a test substance by which the phenotype of Alzheimer's disease in the neuron is not detected or is detected but suppressed is sorted as the substance that inhibits EDIL3 or the active ingredient of the pharmaceutical composition or therapeutic agent for treating Alzheimer's disease. The state in which "the phenotype of Alzheimer's disease in the neuron is not detected" in this case may be determined by a method usually determined by those skilled in the art, and may be, for example, a state in which no qualitative phenotypic difference or no statistically significant quantitative phenotypic difference (e.g., density of dendritic spines) is found between the neuron and a neuron not exposed to EDIL3. In addition, the state in which "the phenotype of Alzheimer's disease in the neuron is detected but suppressed" may be determined by a method usually determined by those skilled in the art, and may be, for example, a state in which the phenotype of Alzheimer's disease (for example, density of dendritic spines) is statistically significantly detected in comparison with a neuron not exposed to EDIL3, but the p value between the neuron and the neuron not exposed to EDIL3 is small as compared with the p value between the neuron and the neuron exposed to EDIL3 in the absence of the test substance, or a state in which the phenotype of Alzheimer's disease is significantly suppressed in comparison with the neuron exposed to EDIL3 in the absence of the test substance (for example, the decrease in density of dendritic spines is significantly suppressed).

A second embodiment of the third aspect of the present disclosure is a method for screening for a substance that inhibits EDIL3, the method including: culturing a cell expressing EDIL3 and a neuron in a medium containing a test substance (co-culturing a cell expressing EDIL3 and a neuron, co-culture step); and detecting a phenotype of Alzheimer's disease from the neuron (detection step). The second embodiment of the third aspect of the present disclosure can be a method for screening for an active ingredient of a pharmaceutical composition or therapeutic agent for treating Alzheimer's disease, the method including: culturing a cell expressing EDIL3 and a neuron in a medium containing a test substance; and detecting a phenotype of Alzheimer's disease from the neuron.

The second embodiment of the third aspect of the present disclosure can be performed in the same manner as the first embodiment of the third aspect of the present disclosure except that EDIL3 to which the neuron is exposed is EDIL3 secreted by the cell expressing EDIL3. That is, the co-culture step in the second embodiment of the third aspect of the present disclosure can be performed in the same manner as the exposure step according to the first embodiment of the third aspect of the present disclosure except that EDIL3 or a culture supernatant containing EDIL3 is not added to the culture environment for the neuron, and that the cell expressing EDIL3 is co-cultured instead. At this time, the period during which the cell expressing EDIL3 and the neuron are co-cultured in the co-culture step is not particularly limited as long as it is a period during which the phenotype of Alzheimer's disease can be detected when co-culture is performed in the same manner in the absence of the test substance, and may be, for example, 1 day to 30 days, 2 days to 15 days, or 3 days to 10 days, and the culture conditions may be conditions usually used by those skilled in the art (for example, 37°C, 5%CO₂, and 100% humidity). In addition, the co-culture in the co-culture step may be performed, for example, by adhering each of the cell expressing EDIL3 and the neuron to a culture vessel in a state of being spatially separated in advance using a culture insert or the like, and then culturing both the cells in the same culture medium through removing the culture insert or the like. The detection step in the second embodiment of the third aspect of the present disclosure can be performed similarly to the exposure step in the first embodiment of the third aspect of the present disclosure.

The cell expressing EDIL3 according to the second embodiment of the third aspect of the present disclosure may be a cell expressing EDIL3 under physiological conditions or a cell in which EDIL3 is overexpressed by transduction thereof. The cell expressing EDIL3 under physiological conditions may be, for example, a glial cell, and the glial cell may be, for example, an astrocyte. The transduction of EDIL3 in the cell in which EDIL3 is overexpressed by transduction thereof can be carried out according to a method commonly carried out by those skilled in the art using materials and reagents commonly used in transduction by those skilled in the art. The cell transduced with EDIL3 as described above is not particularly limited, and may be, for example, a glial cell such as an astrocyte, or may be a cultured cell line (cell line) on which protein transduction is widely and generally performed, such as HEK293 cell, HeLa cell, or NIH3T3 cell.

A third embodiment of the third aspect of the present disclosure is a method for screening for a substance that inhibits EDIL3, the method including: culturing a cell expressing EDIL3 in a medium containing a test substance (contact step); and evaluating expression or secretion of EDIL3 (evaluation step).

As the cell expressing EDIL3 in the third embodiment of the third aspect of the present disclosure, the same cells as the cells expressing EDIL3 according to the second embodiment of the third aspect of the present disclosure can be used. The contact step in the third embodiment of the third aspect of the present disclosure can be performed in the same manner as the exposure step according to the first embodiment of the third aspect of the present disclosure except that the cell expressing EDIL3 is used as the cell instead of the neuron, and that the cell expressing EDIL3 is not exposed to EDIL3 not derived from the cell expressing EDIL3.

The evaluating expression or secretion of EDIL3 in the evaluation step according to the third embodiment of the third aspect of the present disclosure may be, for example, quantifying an mRNA encoding EDIL3, a cDNA complementary to the mRNA, or EDIL3 in the cell expressing EDIL3, a cell disrupted product (lysate) prepared from the cell, or a culture supernatant in which the cell is cultured. As a specific example, it may be quantifying EDIL3 in a medium (culture supernatant in which the cell expressing EDIL3 is cultured). The quantification of EDIL3 in this case may be performed using, for example, an anti-EDIL3 antibody or an EDIL3 binding fragment thereof. The quantifying the mRNA encoding EDIL3, cDNA complementary to the mRNA, or EDIL3 according to the third embodiment of the third aspect of the present disclosure may be performed by the same method as described in the first aspect of the present disclosure.

In the evaluation step according to the third embodiment of the third aspect of the present disclosure, for example, when expression or secretion of EDIL3 in the cell expressing EDIL3 exposed to the test substance (EDIL3-expressing cell to be evaluated) is lower than the expression or secretion of EDIL3 in a cell expressing EDIL3 (control) similarly prepared except that the EDIL3-expressing cell is not contacted with the test substance, the test substance can be sorted as the substance that inhibits EDIL3 . For example, when the amount of mRNA encoding EDIL3, cDNA complementary to the mRNA, or EDIL3 in the EDIL3-expressing cell exposed to the test substance is lower than the amount of mRNA encoding EDIL3, cDNA complementary to the mRNA, or EDIL3 in the control, the test substance can be sorted as the substance that inhibits EDIL3. As an example, when the average value of the amount of mRNA encoding EDIL3, cDNA complementary to the mRNA, or EDIL3 in the EDIL3-expressing cell exposed to the test substance is 0.9 times or less, 0.8 times or less, 0.7 times or less, 0.6 times or less, 0.5 times or less, 0.4 times or less, 0.3 times or less, 0.2 times or less, or 0.1 times or less the average value of the amount of mRNA encoding EDIL3, cDNA complementary to the mRNA, or EDIL3 in the control, the test substance may be sorted as the substance that inhibits EDIL3.

As used herein, the term "comprising" or "including" is intended to be open-ended and inclusive, and does not exclude additional, undescribed characteristics, unless the context clearly dictates otherwise, and include the closed term "consisting of" or "consisting essentially of."

Unless defined differently, all terms (technical and scientific terms) used herein have the same meaning as broadly understood by those skilled in the art to which the present invention belongs. The terms used herein are to be interpreted as having a meaning that is consistent with their meaning in this specification and the related art, and are not to be interpreted in an idealized or overly formal sense, unless a different definition is explicitly stated.

### Examples

Hereinafter, the present disclosure will be described more specifically with reference to examples, but is not limited to the following examples. Hereinafter, induced pluripotent stem cells (iPS cells) are also referred to as iPSCs, and human astrocytes obtained by inducing iPSCs are also referred to as iPasts.

### <Experimental Method>

The examples were carried out according to the experimental methods described below, unless otherwise stated.

### <Test Design>

The purpose was to elucidate detailed molecular mechanisms of astrocyte APOE4-mediated synaptic pathophysiology in Alzheimer's disease (AD). The present inventors first generated human astrocyte cell models (iPasts) induced from isogenic iPSCs expressing APOE3 or APOE4. After confirming that APOE3/3 and APOE4/4 iPasts showed an overall astrocyte profile regardless of genotypic difference, a co-culture system of human iPasts and mouse primary hippocampal neurons was developed using a culture insert to investigate the influence of astrocytic factors on synapse integrity. The hypothesis that APOE4 astrocytic toxic factors induce dendritic spinal degeneration was validated. Two independent clones (#87 and #89) were fully used for isogenic iPSCs carrying APOE4/4 genotype. Sample size was selected based on previous extensive experience with iPast culture. For molecular/biochemical results, at least n = 3 culture batches were used in each experimental genotype. Transcriptome analysis was performed using three culture batches of parental APOE3/3 iPasts and one culture batch of isogenic iPasts carrying each APOE4/4 genotype (#87-5, #87-8, #89-1 and #89-8). All animal experiments were conducted in accordance with the National Institute of Health and Pharmaceutical Science and the Experimental Animal Guidelines of the Ministry of Education, Culture, Sports, Science and Technology, and were approved by the Keio University Institutional Animal Care and Use Committee in accordance with the same guidelines (Approval Number: 15093). Human ethics approval was obtained by the Ethics Committee in Keio University School of Medicine (Approval Number: 20080016).

### <Culture of Undifferentiated iPSCs>

The healthy control human iPSC line 201B7 (female Caucasian, 36-years old) was cultured in StemFit/AK02N (Ajinomoto) as feeder-free cultures. iPSCs were passaged by 0.5 × TrypLE select (Thermo Fisher Scientific) every 7 days and seeded at 1.5 × 10⁴ cells/well in 6-well plate coated with 1.5 µg/ml iMatrix-511 silk (Laminin-511 E8, Nippi) in the presence of 10 µM Y27632 (Nacalai). Media were changed every two days. For on-feeder iPSC cultures, cells were maintained on mitomycin C-treated SNL murine fibroblast feeder cells in human ESC medium: Dulbecco's Modified Eagle Medium: Nutrient Mixture F-12 (DMEM/F12, Sigma) containing 20% KnockOut serum replacement (KSR, Life Technologies), 0.1 mM nonessential amino acids (NEAA, Sigma), 0.1 mM 2-mercaptoethanol (Sigma), and 4 ng/ml fibroblast growth factor 2 (FGF-2, PeproTech) in an atmosphere containing 3% CO₂.

### <Statistical Analysis>

Data are presented as the mean value ± standard error of the mean (Mean ± S.E). Statistical significance was defined as p < 0.05. The data were analyzed using R version 4.0.0 (The R Foundation) or Prism software (GraphPad Software, Ver. 8.2.0).

### <Construction of APOE Targeting Vectors for Gene Editing>

To construct a targeting vector for APOE allele, a 3.36 kb genomic DNA, which contains the exons 3-4, was first amplified by PCR using genomic DNA of ax7111 iPSC carrying APOE4/4 as a template, with a primer pair 5'-GGGCTGCGTTGCTGGTCACATT-3' (SEQ ID NO: 27) and 5'-ACCCCATCTCCACACACACCAC-3' (SEQ ID NO: 28), and subcloned into pCR-Blunt II-TOPO vector (Invitrogen). After sequence confirmation, a 1.10 kb left-arm fragment (L1) encompassing the third exon of APOE gene, a 1.23 kb right-arm fragment (R1) and a 0.47 kb right-arm fragment (R2), in which the combined right-arm encompasses the fourth exon of APOE gene, were amplified by PCR using the above plasmid as a template. The PCR primers used were 5'-CAACTTTGTATAATAAAGTTGAGTGCAGTGGCGGGATCTCG-3' (SEQ ID NO: 11) and 5'-ATGATTATCTTTCTAGGGTTAACGACAGGGGCAGAATGAAACC -3' (SEQ ID NO: 12) for the left arm L1, 5'-CAGACTATCTTTCTAGGGTTAACTGGGTCTCTGCTGGTTCTAG C-3' (SEQ ID NO: 13) and 5'-CGTGGGATCCCATGGCTGCAGGCTTCGGCGTTC-3' (SEQ ID NO: 14) for the right arm R1, and 5'-CCATGGGATCCCACGCCACCCCGTGCCTCCTG-3' (SEQ ID NO: 15) and 5'-CAACTTTGTATAGAAAAGTTGAGGAGTTTGAGACCTGCCTGG-3' (SEQ ID NO: 16) for the right arm R2. These genomic fragments were used as 5'-(L1) and 3'-(R1 and R2) homologous regions in APOE target vector. L1 fragments were subcloned into HpaI sites in the pENTR2-L3-HpaI-PBL-R1 plasmid (#403) using the In-Fusion HD Cloning Kit (Takara Bio). Both R1 and R2 fragments were subcloned into HpaI sites in the pENTR2-R2-PBR-HpaI-L4 plasmid (#404) using the In-Fusion HD Cloning Kit. A targeting plasmid was constructed by modified Multisite Gateway-based method according to Non Patent Literature 11. These plasmids bearing 5'- or 3'-targeting arm were confirmed by Sanger sequencing and assembled into a backbone vector (#0042, pUC-DEST-R3R4) with a selection marker cassette plasmid (#0102, pENTR-L1-PGK-puroΔTK-L2) using the Gateway LR Clonase II Enzyme mix (Thermo Fisher Scientific). To select the iPSC clones carrying the targeted alleles, the selection marker cassette plasmid contained a puroΔTK (a fusion protein of a puromycin N-acetyltransferase and a truncated version of herpes simplex virus type 1 thymidine kinase) cassette flanked by two Inversed terminal repeat (ITR) sequences to allow removal of the puroΔTK gene by piggyBac transposase.

To generate a plasmid expressing single guide RNA (sgRNA) for APOE genome editing, the following oligonucleotides were annealed and inserted into a BbsI site of pSpCas9(BB)-2A-GFP (Addgene, PX458). This site can simultaneously express S. pyogenes Cas9 (SpCas9) and GFP. 5'-CACCGGCCCCCCAAGACTTAGCGAC-3' (SEQ ID NO: 17) and 5'-AAACGTCGCTAAGTCTTGGGGGGCC-3' (SEQ ID NO: 18) were used as oligonucleotides for APOE sgRNA1, and 5'-CACCGCGGGGTGGCGTGGGGTCGCA-3' (SEQ ID NO: 19) and 5'-AAACTGCGACCCCACGCCACCCCGC-3' (SEQ ID NO: 20) were used as oligonucleotides for APOE sgRNA2.

### <Generation of Isogenic iPSCs Expressing APOE4/4>

To generate iPSCs carrying the targeted APOE4/E4 alleles, 201B7 iPSCs were electroporated with a targeting vector along with two sgRNA-expressing plasmids. Puromycin was applied to the culture at 1.0 µg/ml 48 h later and the surviving iPSC clones were picked following puromycin selection. Genomic DNAs around the targeting region of total 36 iPSC clones were first amplified by PCR using the primer pair for APOE genomic cloning as shown above, which encompasses both homologous recombination arms. Next, nested PCR was performed using primer pair 5'-AAGAAGCATTTGTGGAGCACC-3' (SEQ ID NO: 21) and 5'-TGAATCTTTATTAAACTAGG-3' (SEQ ID NO: 22), which encompasses two sgRNA target sequences. Nine iPSC clones were positive for proper homologous recombination in both alleles, giving rise to a 4795 bp bands, which represent the targeted allele. These homozygous clones were further analyzed by cutting with a de novo BamHI site at the sgRNA2 sequence. Three out of 9 iPSC clones were confirmed to carry the de novo BamHI site at both alleles. Further, APOE4/4 was verified by sequencing the targeted alleles in 2 clones (#87 and #89). To generate iPSCs bearing genuine APOE4/4 alleles, the selection cassette was removed by introducing a plasmid expressing excision-only piggyBac transposase (PBx) into the established targeted clones and selected under 10 µM ganciclovir treatment for 10 days. Finally, two successful APOE4/4 clones were obtained from each targeted clones (#87-5, #87-8, #89-1, #89-8).

### <iPSC-derived Astrocyte (iPast) Induction>

iPSC colonies were cultured one night in suspension without FGF-2 and with 10 µM Y-27632 (Nacalai, San Diego, CA, USA) to form embryoid bodies (EBs) in a humidified atmosphere of 3% CO₂. From next day, EBs were suspended in EB medium (DMEM/F12 containing5% KSR, 1% L-Glutamine 0.8% NEAA, 0.1 mM 2-mercaptoethanol and Penicillin/Streptomycin) to enhance the differentiation into neural lineage. The medium contained: Days 1 to 3: 3 µM SB431542 (Sigma) and 3 µM CHIR99021 (Focus Biomolecules); Days 4 to 7: 1 µM retinoic acid (Sigma); and Days 7 to 16: 1 µM retinoic acid and 1 µM purmorphamine (Cayman, Tokyo, Japan). At day 16, EBs were dissociated for suspension culture at a density of 1 to 4 × 10⁵ cells/mL in neurosphere medium consisting of media hormone mix (MHM) supplemented with 2% B-27 (Nacalai), 0.8% NEAA, 1 µM purmorphamine, 20 ng/mL FGF-2, 10 ng/mL epidermal growth factor (EGF, PeproTech) in a humidified atmosphere of 5% CO₂. The medium was changed every 4 to 6 days to form primary neurospheres. At day 32, primary neurospheres were dissociated and cultured at a density of 1 to 4 × 10⁵ cells/mL in neurosphere medium without purmorphamine. At day 48, secondary neurospheres were dissociated and cultured at a density of 4 × 10⁵ to 1 × 10⁶ cells/well in 6-well plates coated with Matrigel (Corning) in iPast differentiation medium consisting of MHM supplemented with 2% B-27, 0.8% NEAA, 10 ng/mL of brain-derived neurotrophic factor (BDNF, R&D), 10 ng/mL of glial cell-derived neurotrophic factor (GDNF, Alamone) in a humidified atmosphere of 5% CO₂. The medium was changed every 7 days. At almost confluent culture, iPasts were dissociated into single cells using accutase (nacalai) and the dissociated cells were passaged at a density of approximately 1 × 10⁶ cells/well in 6-well plates coated with Matrigel.

### <iPast Culture and A1 Reactive Induction>

iPasts were maintained in iPast-differentiation medium unless otherwise mentioned. iPast cultures were harvested for analysis during moderate number of passages 7 to 15. iPasts were also stored in serum-free CellBanker(registered trademark) 2 (Zenoaq). A1-like phenotype was induced by treating iPasts with TIC cocktail (30 ng/ml TNFα (AF-300-01A, Peprotech), 3 ng/ml IL-1α (200-01A, Peprotech), 400 ng/ml C1q (PRO-554L, Prospec)) in DMEM/F12 medium for 24 hrs.

### <Karyotype Analysis>

G-banding analysis of the iPSC lines was conducted by the LSI Medience Corporation (Tokyo, Japan).

### <Quantitative Reverse Transcription-PCR (qRT-PCR)>

Total RNA was purified with RNeasy Mini Kit (QIAGEN) and reverse-transcribed in the presence of random hexamers. qPCR reactions were performed using TB Green Premix Ex Taq II (TAKARA BIO Inc.) in ViiA7 System (Applied Biosystems) with cDNA and gene-specific primers. Analysis was performed with at least three independent cultures and threshold cycle (Ct) values of interest were normalized to those of GAPDH. The sequences of the primers used for EDIL3 and GAPDH are as follows:
EDIL3:5'-AGCATACCGAGGGGATACATT-3' (SEQ ID NO: 9) and 5'-CAAGGCTCAACTTCGCATTCA-3' (SEQ ID NO: 10)
GAPDH:5'-AGGCTGAGAACGGGAAGCTT-3' (SEQ ID NO: 23) and 5'-ACTCCACGACGTACTCAGCG-3' (SEQ ID NO: 24)

### <Construction of Expression Plasmid>

For plasmid expressing human EDIL3 under the CAGGS promoter, PCR product was first subcloned into XbaI-EcoRI of pCAGGS vector and then verified by sequencing. Human EDIL3 cds fragments were amplified from human brain cDNAs (1446 base pairs) using specific primers having the sequences: 5'-ATTTCTAGAATCATGAAGCGCTCGGTAGC-3' (SEQ ID NO: 25) and 5'-TAAGAATTCTCATTCCTCCTCTGTGCAGC-3' (SEQ ID NO: 26).

### <Western Analysis>

Cultured cells were homogenized in RIPA buffer (50 mM Tris-Cl (pH 7.6), 150 mM NaCl, 0.5 mM EDTA, 1% NP40, 0.5% sodium deoxycholate, 0.1% SDS, Complete protease inhibitor cocktail (Roche), 1 mM PMSF). Equal amount of cell lysate (10 to 20 µg per lane) or culture medium were separated in Extra PAGE One Precast Gel (Nacalai tesque Inc.) and transferred to PVDF membranes. The membranes were blocked in 5% nonfat milk/TBS for 1 hour and incubated with specific primary antibodies shown as below: goat anti-ApoE (1:1000, AB947, Millipore), rabbit anti-EDIL3 (1:1000, ab190692, abcam), mouse anti-actin (1:10000, A1978, Sigma). The membrane was then incubated with IRDye 800CW or IRDye 680-labeled secondary antibodies (LI-COR Bioscience). Signals were developed and quantified with an Odyssey Infrared Imaging System (LI-COR Bioscience).

### <Immunocytochemistry>

iPast or iPSC cultures in multi-well plate were fixed with 4% paraformaldehyde, blocked with a solution containing 5% FBS and 0.1% Triton X-100 for 1 h at room temperature, and incubated with the indicated primary antibodies overnight at 4°C. The following primary antibody was used: rat anti-GFAP (1:500, #13-0300, Thermofisher), mouse anti-S100β (1:500, S2532, Sigma), mouse anti-CD44 (1:200, MA5-13890, Thermofisher), rabbit anti-MAP2 (1:200, AB5622, Millipore), or mouse anti-SSEA-4 (1:500, abcam, ab16287). Cultures were then washed in phosphate-buffered saline and incubated with appropriate Alexa Fluor (405, 488, 555 or 647)-conjugated secondary antibodies (Invitrogen) for 1 h at room temperature. Signals were detected by LSM 700 confocal microscope (Carl-Zeiss).

### <Enzyme-linked Immunosorbent Assay (ELISA) for APOE>

iPast cultures were maintained in 12-well plate for 3 to 7 days. The collected medium was centrifuged to remove insoluble material and stored at -80°C until analysis. The remaining cells were lysed in RIPA buffer and protein concentration was measured by BCA Protein assay (Pierce). 96-well plates were coated overnight with an ApoE antibody (AB947, Millipore) in 0.1 M carbonate buffer at 4°C overnight. The plates were blocked with 1% nonfat milk in PBS, and then washed 3 times with PBST. Recombinant apoE3 (PeproTech) were used as standards for ELISA quantification. Samples were appropriately diluted and incubated at 4°C overnight. The plates were washed and incubated with biotin-conjugated goat anti-ApoE antibody (1:10,000, Meridian Life Science) for 2h at room temperature. After incubation with streptavidin-horseradish peroxidase (1:1,000, Fitzgerald Industries) for 90 min at room temperature, the plate was developed for ~5 minutes by adding tetramethylbenzidine Super Slow substrate (T5569, Sigma). The reaction was stopped with 1 M H₂SO₄ and read at 450 nm with iMark microplate reader (Bio-Rad). Each APOE concentration was normalized by protein levels of the culture.

### <Size Exclusion Chromatography (SEC)>

Approximately 8 ml of the iPast culture media in 10-cm dish with 7-days incubation was concentrated using Amicon Ultra Ultracel-30K (30 kDa cutoff, Millipore). The concentrated medium (~0.7 ml) was loaded onto a Superdex 200 Increase 10/300 GL column (GE Healthcare). The column was developed with phosphate buffer containing 50 mM sodium phosphate (pH 7.4) and 150 mM NaCl at a flow rate of 0.5 ml/min. The effluent was monitored at 280 nm and every 0.5-ml fractions were collected. A small aliquot of each fraction was analyzed by ELISA as described above.

### <Immunoprecipitation of APOE Particles>

Streptavidin-conjugated agarose beads (Sigma) were incubated with biotin-conjugated goat anti-ApoE antibody (K74180B, Meridian Life Science) for 2 h at room temperature with shaking. The anti-ApoE-agarose beads were mixed with the iPast conditioned medium at 4°C overnight. After washing, the agarose beads were re-suspended in 100 µl of TBS with 0.1% Triton X-100. The amounts of cholesterol dissolved in the supernatant were measured by Amplex Red cholesterol assay (Invitrogen). Moreover, the beads were suspended in 100 µl of 0.1 M glycine buffer (pH 2.5), immediately followed by neutralization with Tris-HCl (pH 8.8). The amount of APOE in the mixture was measured by APOE ELISA. The amount of APOE-associated cholesterol was determined by dividing total cholesterol quantities with total APOE quantities in the eluent.

### <Intracellular Lipid Staining>

For the measurement of neutral lipid accumulation, iPasts were plated at 1 × 10⁴ cells/well on the 96-well microplate µCLEAR (greiner, Cat# 655096) coated with 5% Matrigel (Corning) in PBS. iPasts were incubated in DMEM/F12 supplemented with 2% FBS (Sigma) for 2 weeks. iPasts were treated with dimethyl sulfoxide (DMSO) or oleic acid (OA) (final 200 µM) in the medium for 16 hr at 37°C. For staining of neutral lipids, iPasts were treated with LipiBlue (final 0.1 µM, DOJINDO), Calcein-AM (final 1 µg/mL, DOJINDO), DRAQ5 (final 5 µM, Cosmo Bio) in the medium for 1 hr at 37°C. Then, iPasts were washed with DMEM/F12 twice and fixed with 4% paraformaldehyde in PBS for 16 hr at 4°C. The fixed cells were imaged with IN Cell Analyzer 6000 (Cytiva); a set of 4×4 fields was collected from each well using the 20× objective lens. The images were analyzed with In Cell Developer Toolbox version 1.9.2 (Cytiva). The round shape lipid particles stained by LipiBlue in the Calcein-AM-positive cells were counted. The round or oval shape nuclei stained by DRAQ5 were counted for the identification of the number of cells.

### <Mouse Primary Hippocampal Neuronal Cultures>

Hippocampi from wild-type C57BL/6J pups were dissected and treated with 0.25% trypsin at 37°C for 12 min. Cells were plated at a density of 65000 cells/cm² on poly-D-lysine (Sigma)-coated 12-mm coverglass (Costar). Primary hippocampal neurons were cultured in Neurobasal medium (Thermo Fisher Scientific) supplemented with 1% B27 and 1× GlutaMAX until Days 16 to 21 of culture (DIV 16 to 21) for further molecular and morphological analyses.

### <Neuronal Morphology Analysis>

Primary neurons were sparsely transfected at the timing of Days 5 to 6 of culture (DIV 5 to 6) with a plasmid expressing actin-EGFP (a kind gift of Dr. Bito) together with one expressing DsRed protein using Lipofectamine 2000 (Thermo Fisher Scientific). For the last 4 to 6 days, approximately 1 × 10⁵ cells of iPasts were co-cultured using Transwell 0.4 µm Membrane Inserts (Corning). Cultures were fixed with 4% paraformaldehyde, permeabilized, blocked, and incubated with chicken anti-GFP (1: 1000, aveslab, GFP-1010) and rabbit anti-RFP (1:500, MBL, PM005) antibodies overnight at 4°C. The sample was then washed and incubated with appropriate Alexa Fluor 488 or 555-conjugated secondary antibodies (Invitrogen) for 1 h at room temperature. After wash with PBS, the coverglass was mounted on slidegrass with a ProLong Gold AntiFAde ReAgent (Invitrogen). Images were taken on the GFP-positive neurons by LSM 700 confocal microscope with 63× lens. Each of these images was acquired in a z-stack at 0.5-µm interval, on average 6 to 8 slices per z-stack. The number of spines on secondary or tertiary neurite was counted with ImageJ software (NIH) and calculated the spine density by dividing the spine number with neurite length. DsRed signals were acquired by the confocal microscope with 10× lens. Neurite complexity was analyzed using Sholl analysis of ImageJ plugins.

### <Mass Spectrometry Analysis>

iPasts were maintained in the same iPast-differentiation medium, except a usage of phenol red-free one. Proteins in conditioned media were denatured with 2M urea in 100 mM Tris-HCl pH 8.5 by ultrasonication on ice for 10 min, reduced and alkylated with 10 mM tris(2-carboxyethyl)phosphine (Thermo Fisher Scientific) and 40 mM 2-chloroacetamide (FUJIFILM Wako), and digested with 0.5 µg LysC and 0.5 µg trypsin overnight. Resulting peptides were purified using a reversed-phase SDB-XC StageTip. After TMT (tandem mass tag) labeling, labeled peptides were fractionated into nine fractions using a high pH reversed-phase peptide fractionation kit (Thermo Fisher Scientific) with elution solutions containing the following concentration of ACN: 10%, 12 5%, 15%, 17 5%, 20%, 22.5%, 25%, 35%, and 60%.

An LC/MS/MS system including an UltImate 3000 RSLCnano pump (Thermo Fisher Scientific) and an Orbitrap Fusion Lumos tribrid mass spectrometer (Thermo Fisher Scientific) equipped with the FAIMSpro interface (Thermo Fisher Scientific) was employed. All fractions were divided into four parts and analyzed by four measurements with different FAIMS compensation voltages of -40, -55, -70, and -85. Peptides were injected by an HTC-PAL autosampler (CTC Analytics), loaded on a 15 cm fused-silica emitter packed with 3 µm ReproSil-Pur C18-AQ (Nikkyo Technos), and separated by the following linear gradients (solvent A, 0.1% formic acid; solvent B, 0.1% formic acid in 80% ACN): for fractions 1 to 4, 5 to 10% B in 5 min, 10 to 30% B in 70 min, 30 to 99% B in 5 min, and 99% B for 10 min; for fractions 5 to 9, 5 to 15% B in 5 min, 15 to 35% B in 70 min, 35 to 99% B in 5 min, and 99% B for 10 min. The flow rate was set to 300 nL/min. Peptides were ionized at 2,000 V. All MS1 spectra were acquired over the range of 400 to 1600 m/z in the Orbitap analyzer with the resolving power of 120000. For the subsequent MS/MS analysis, precursor ions were selected and isolated in top-speed mode (cycle time = 3 sec, isolation window = 1.4 m/z), activated by collision-induced dissociation (collision energy = 35), and detected in the ion trap analyzer in turbo mode. For TMT-reporter ion quantification, MS3 scans were performed in synchronous precursor scan mode by isolating the top 10 intense fragment ions. In all scans, maximum injection time was set to "auto". In MS and MS2 scans, the AGC target value was set to "standard", while that was set to 250% in MS3 scans. Dynamic exclusion time was set to 30 sec.

LC/MS/MS raw data were processed using MaxQuant (v.1.6.17.0). Database search was implemented against the UniprotKB/SwissProt (March, 2019) human database including isoforms, concatenated with human APOE4 sequence. The following parameters were applied: 10-plexed TMT quantification at the MS3 level, precursor mass tolerance of 4.5 ppm, fragment ion mass tolerance of 0.5 Da, and minimal peptide length of 7 amino acids. Cysteine carbamidomethylation was set as a fixed modification, while methionine oxidation and acetylation on the protein N-terminus were allowed as variable modifications. False discovery rates were estimated by searching against a reversed decoy database and filtered for < 1% at the peptide-spectrum match and protein level. TMT-reporter intensities were log2-converted and normalized by the trimmed mean of M values method.

### <Transcriptome Analysis>

Total RNA was purified with RNeasy Mini Kit or RNeasy Micro Kit (QIAGEN). RNA quality was assessed using an Agilent 2100 Bioanalyzer (Agilent Technologies, Palo Alto, CA, USA). Total 500 ng of RNA was reverse-transcribed, biotin-labeled, and hybridized onto Clariom S Array (Affymetrix) following the protocol detailed by Affymetrix. Each data was acquired from APOE3/3 (201B7, n = 3 independent culture lots) and APOE4/4 (#87-5, #87-8, #89-1, #89-8, n = total 4 lines) iPasts. Raw data were normalized using Robust Multichip Analysis (RMA) algorithm. PCA and clustering heatmap were analyzed with differentially expressed genes by logFC > 0.5 or logFC < -0.5 (FDR p-val < 0.05).

### <Knockdown of EDIL3>

The plasmids for lentivirus production used were: pENTR4-H1, pCS-RfA-CG, pCMV-VSV-G, and pCAG-HIVgp. These plasmids were provided by RIKEN BioResource Research Center (RIKEN BRC) through the National Bioresource Project (cat. RDB04395, RDB04390, RDB04392 and RDB04394) of the Ministry of Education, Culture, Sports, Science and Technology, Japan. Knockdown lentiviral shuttle vectors were generated by inserting siRNA oligo-DNA into donor plasmid pENTR4-H1. The target sequence (5'-GTGGAATATGTACAGATCT-3', SEQ ID NO: 29) of shRNA designed for human EDIL3 mRNA in the shuttle vector for knockdown and non-functional shRNA (5'-TAAGGTTAAGTCGCCCTCG-3', SEQ ID NO: 30) in the negative control were each recombined into lentiviral shuttle plasmid pCS-RfA-CG using the Gateway system. All constructs were confirmed by Sanger sequencing. These shuttle plasmids were packaged into self-inactivating lentiviruses according to the method described in Non Patent Literature 13.

### <Preparation of human brain tissue>

Human postmortem brain tissues were obtained from the Brain Bank for Aging Research (BBAR) at Institute of Gerontology in Tokyo Metropolitan Geriatric Hospital and Institute of Gerontology under the approval of the Institutional Review Board (Approval Number: R21-125). All AD cases met the Consortium to Establish a Registry for Alzheimer's Disease (CERAD) criteria for clear diagnosis of AD. The grey matter [BA=39/40] of the parietal lobes of four AD patients and two healthy controls was analyzed.

### <Immunohistochemical Staining and Quantitative Analysis of Human Brain Tissue>

Brain tissues were fixed with 4% paraformaldehyde, embedded in paraffin wax, and cut into 6 µm thick sections. After deparaffinization, the brain sections were immersed in a target retrieval solution (S1699, DAKO) and boiled in a microwave oven for 10 minutes. After blocking with PBS containing 3% goat normal serum and 1% bovine serum albumin, the sections were incubated with rabbit anti-EDIL3 antibody (1:200, #12580-1 - AP, Proteintech), rat anti-GFAP antibody (1:500, #13-0300, Thermofisher) and mouse anti-Aβ antibody (1:200, 12B2, IBL) in a blocking solution overnight at 4°C. The sections were then washed three times for 5 min in PBS, followed by incubation for 2 h in the dark with Alexa Fluor (488, 555, or 647)-conjugated secondary antibodies (Invitrogen) in PBS containing 3% goat normal serum at room temperature. Nuclei were counterstained with Hoechst33342. Fluorescence signals were detected by LSM 700 confocal microscope (Carl-Zeiss).

Histological analysis was performed by the method described below. A concentric ring was set outside amyloid plaque using ImageJ to increase the radius from the amyloid plaque by 10 µm. The number of astrocyte (cells positive for both GFAP and Hoechst 33342) was counted manually for each concentric ring, and the astrocyte ratio was calculated by dividing the number of astrocytes by the total number of cells (Hoechst 33342 positive nuclei). For EDIL3 accumulation levels, EDIL3 fluorescence intensity was calculated for plaque region and radiocentric rings. In each group, at least a total of 13 plaques in the postmortem brains of 2 AD patients were analyzed in each group.

### <Example 1: Preparation of isogenic iPSC-derived astrocytes carrying APOE4/4 variant>

Isogenic iPSC-derived astrocytes carrying APOE4/4 variant were prepared according to the method described in the Experimental Method section.

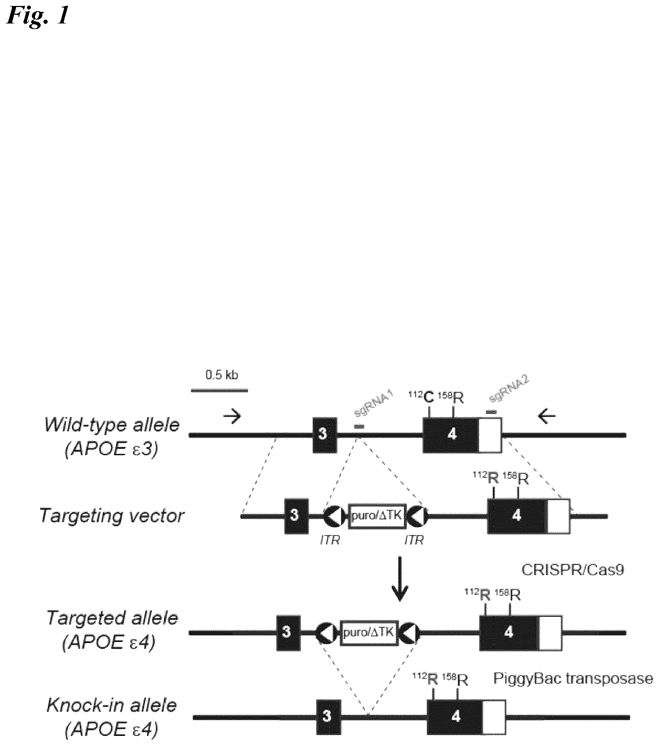

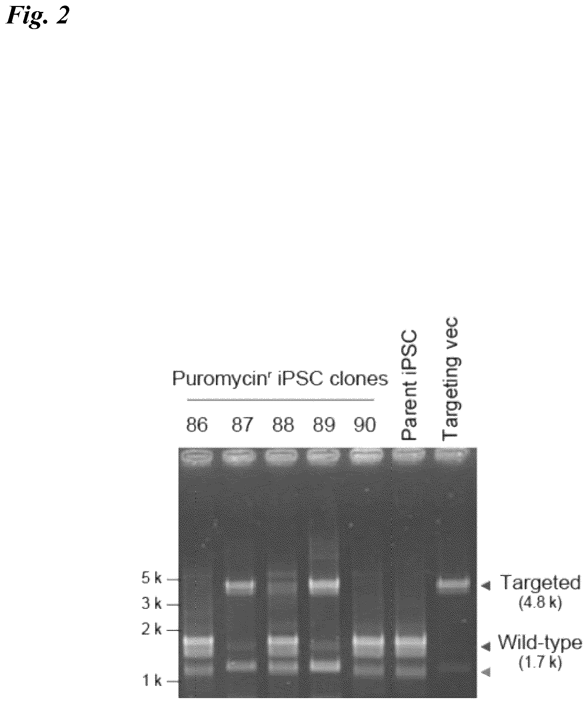

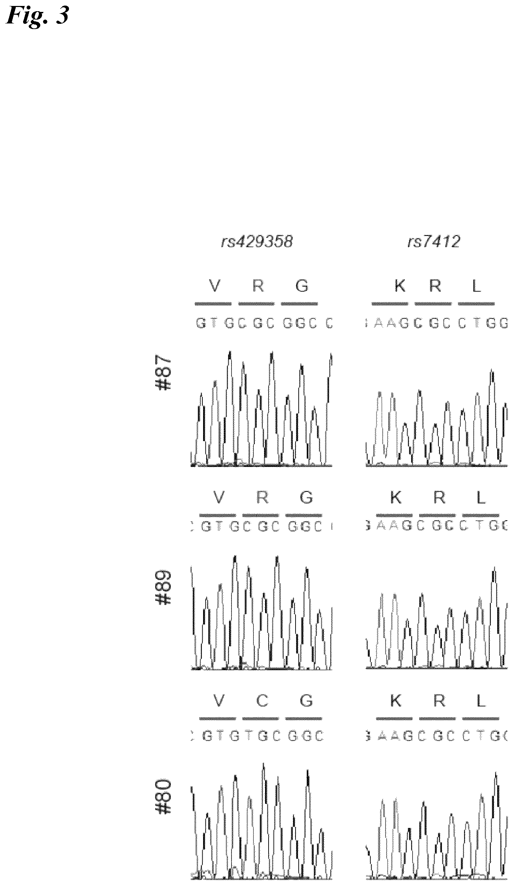

First, isogenic iPSCs carrying APOE4/4 variant were prepared. FIG. 1 shows an outline of a method for generating an allele in which a gene encoding APOE4 was knocked-in to an APOE3 allele (Wild-type allele). FIG. 2 shows results of gel electricity analysis after amplifying genes of five iPSC clones (#86 to #90) into which the generated allele was introduced by PCR. Bands of molecular weight corresponding to the targeting vector used for generation were detected in the two clones #87 and #89 out of the five clones #86 to #90. Furthermore, as shown in the sequencing results shown in FIG. 3, the 112th amino acid in #80, which is a control iPSC having homozygous APOE3/3 genotype, was cysteine, whereas the 112th amino acid in the two clones #87 and #89 was arginine, which corresponds to the sequence of APOE4. From these results, the two clones #87 and #89 were selected as iPSCs having APOE4/4 genotype.

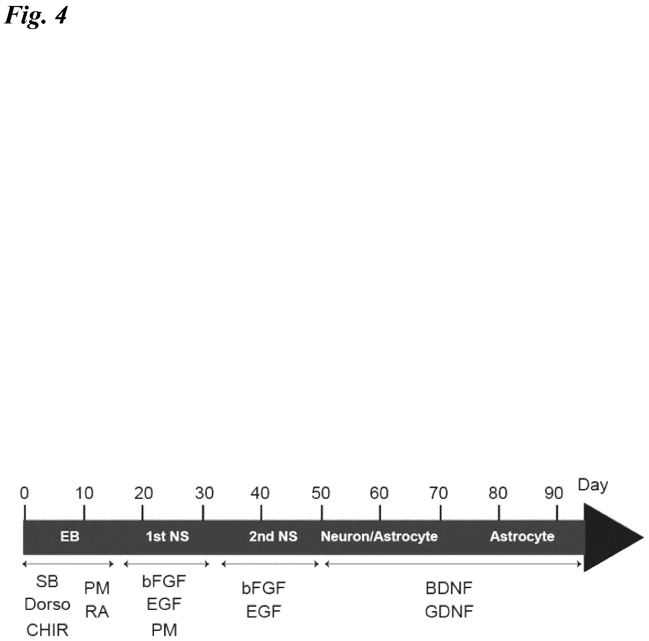

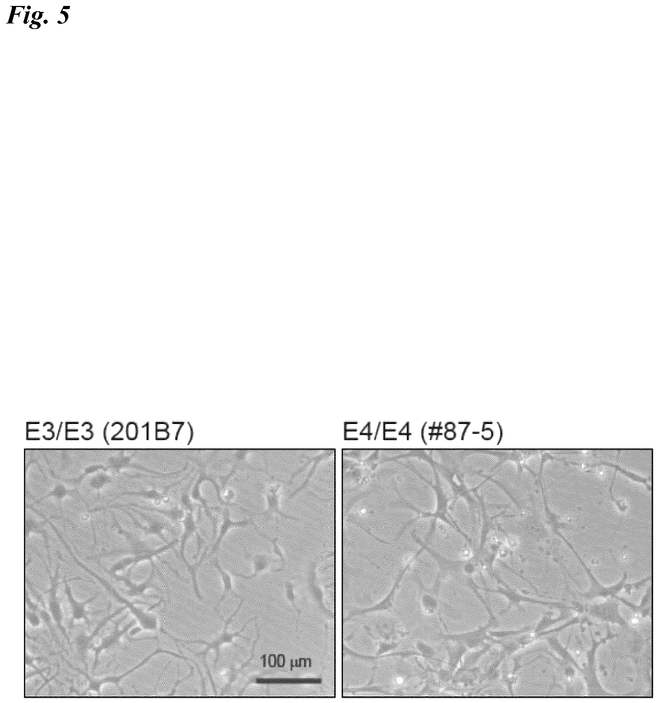

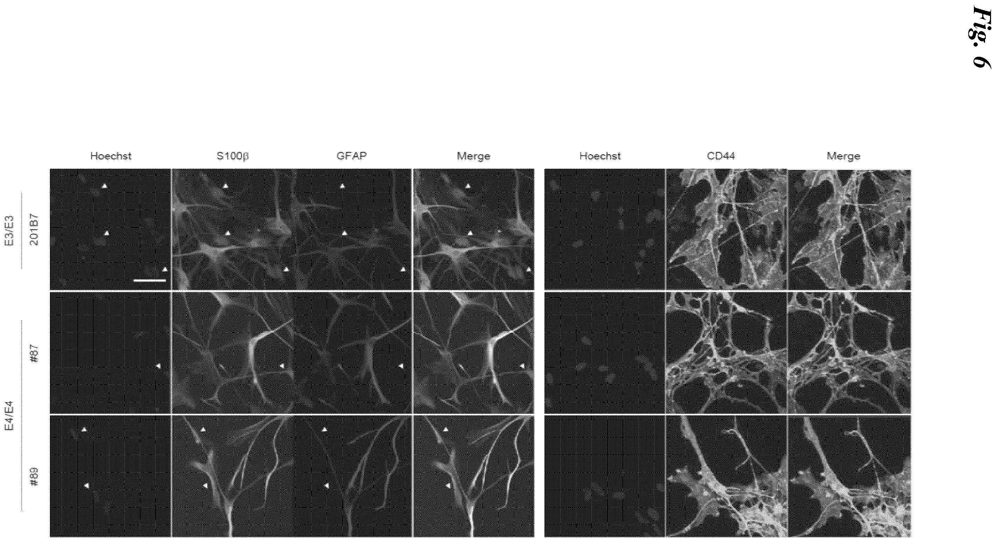

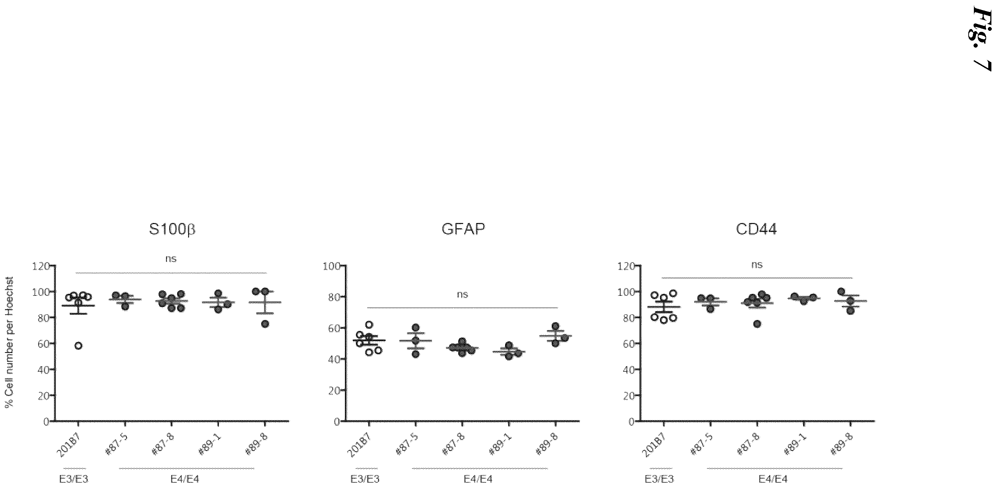

iPSCs having APOE4/4 genotype were then induced into astrocytes. FIG. 4 shows an outline of an outline of a method for inducing iPSCs into astrocytes. FIG. 5 shows representative morphologies of an astrocyte (E4/E4 (#87-5)) derived from the iPSC having APOE4/4 genotype and an astrocyte (E3/E3 (201B7)) derived from 201B7 as the iPSC having APOE3/3 genotype, which were obtained through such an induction process. In addition, for the astrocytes (iPasts) obtained through such an induction process, FIG. 6 shows fluorescence images when astrocyte markers S100β, GFAP, and CD44 were immunofluorescently stained and nuclei were stained with Hoechst, and FIG. 7 shows fluorescence intensities of S100β, GFAP and CD44 in the fluorescence images (Mean ± S.E, n = 3 to 6, ns: Not Significant). As shown in the above, the cells induced into astrocytes from the iPSC having APOE4/4 genotype showed the same morphology and expression of the markers as those of the cells induced into astrocytes from 201B7 as the iPSC having APOE3/3 genotype, revealing that the iPSCs having APOE4/4 genotype were induced into astrocytes.

### <Example 2: Study on Expression Level of apoE Protein in Astrocyte Derived from iPSC Having APOE4/4 Genotype>

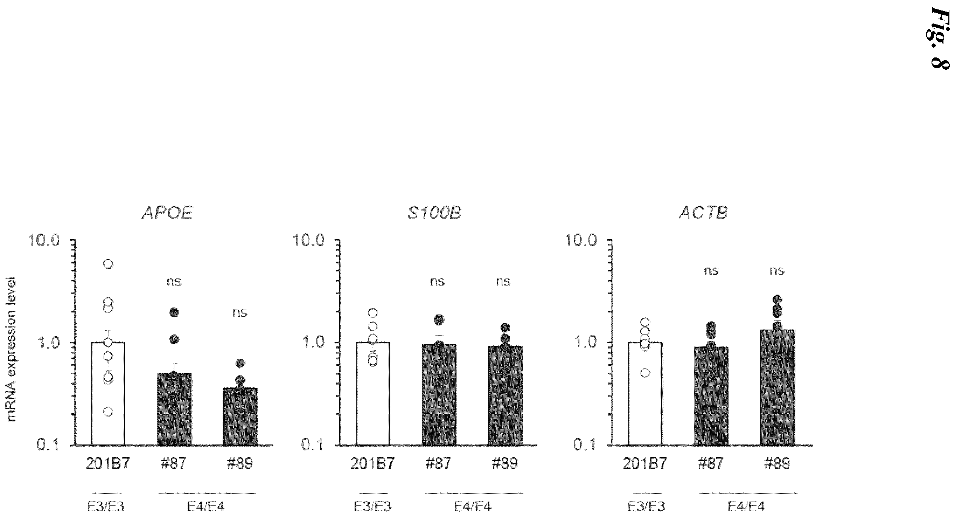

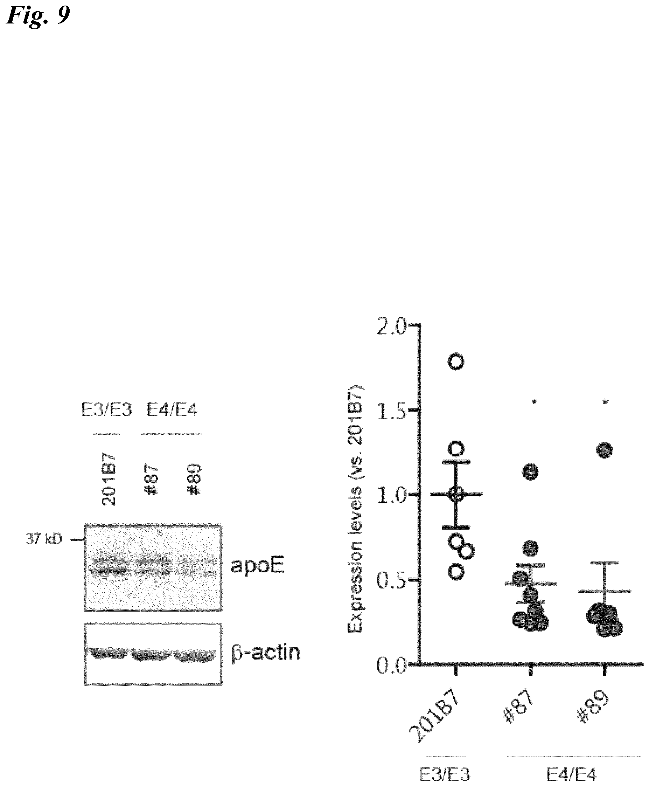

For the astrocytes (iPast) derived from the iPSCs having APOE4/4 genotype generated in Example 1, the gene amounts and expression levels of various proteins including APOE protein were examined by the method described in the Experimental Method section. FIG. 8 shows results of measuring the expression levels of mRNAs encoding APOE protein (APOE), the astrocyte marker S100β, and β actin (ACTB). According to FIG. 8, no significant difference in expression level of the mRNA encoding APOE protein was found between the iPasts having APOE4/4 genotype and the iPasts having APOE3/3 genotype. On the other hand, according to the results of quantifying the expression levels of APOE protein and β-actin expressed by each iPast by Western blotting shown in FIG. 9, the expression level of APOE protein in the iPasts having APOE4/4 genotype was reduced to about 50% of that of the iPasts having APOE3/3 genotype, and the difference in expression levels of both was significant (in FIG. 9, * indicates that the p value is less than 0.05).

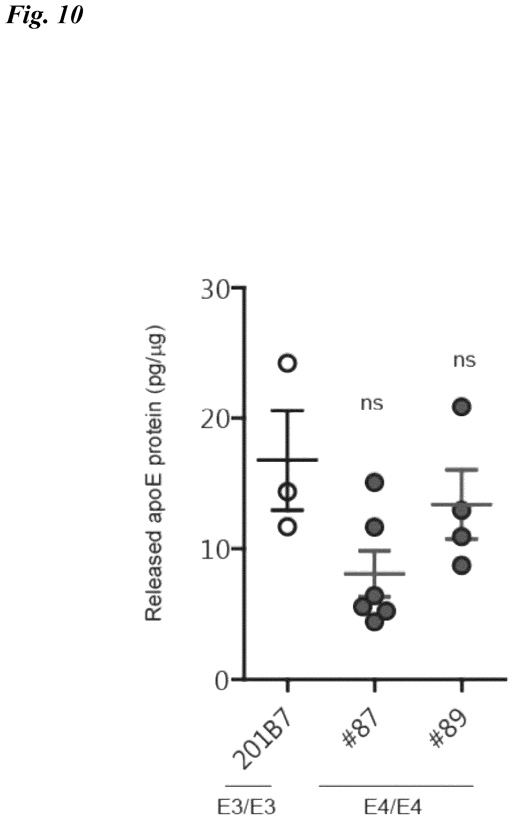

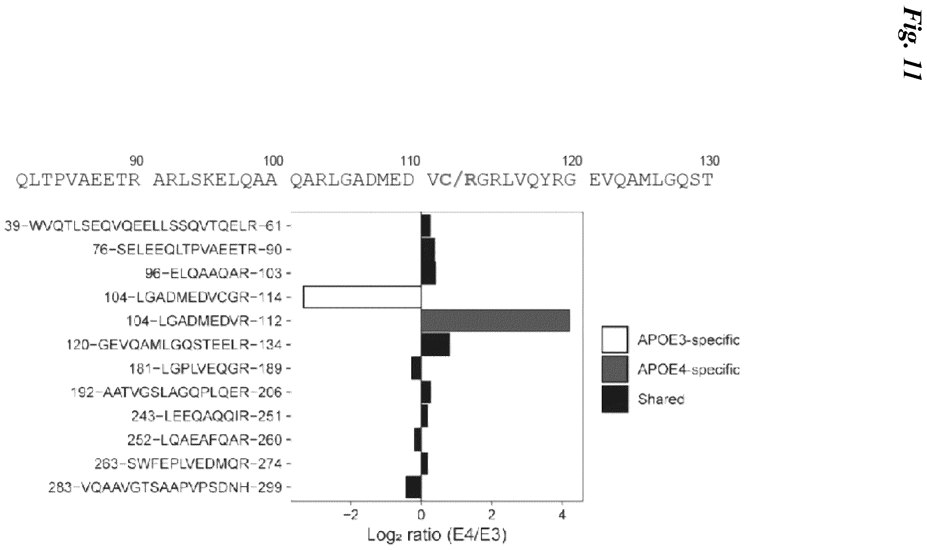

Subsequently, since APOE is a protein secreted extracellularly, the amount of APOE in the medium in which each iPast was cultured was investigated. The results of investigating APOE amount by an ELISA method are shown in FIG. 10. According to FIG. 10, the amount of APOE secreted by the iPasts having APOE4/4 genotype was low, although not significantly different from the amount of APOE secreted by the iPasts having APOE3/3 genotype. Specifically, the amount of APOE secreted by the iPasts derived from 201B7 was 16.78 ± 3.82 ng/µg, the amount of APOE secreted by the iPasts derived from the #87 clone was 7.36 ± 1.75 ng/µg, the amount of APOE secreted by the iPasts derived from the #87 clone was 13.37 ± 2.65, the p value between the iPasts derived from 201B7 and the iPasts derived from the #87 clone in the Dunnett's test was 0.06, and the p value between the iPasts derived from 201B7 and the iPasts derived from the #89 clone was 0.59. As shown in the results of mass spectrometry analysis of the fragments obtained by treating APOE with trypsin shown in FIG. 11, the fragments derived from APOE3 were mutually exclusively detected from the extra-culture fluid of iPasts having APOE3/3 genotype, and the fragments derived from APOE4 were mutually detected from the extra-culture fluid of the iPast having APOE4/4 genotype. From the above, it was shown that the amount of secreted APOE is lower in the iPasts having APOE4/4 genotype than in the iPasts having APOE3/3 genotype, though not significant.

### <Example 3: Study on Neuronal Morphological Change in Neuron Co-cultured with iPast>

Based on the results of Example 2, in order to elucidate the influence of a secretion by iPasts on the morphology of neurons, iPasts having APOE4/4 genotype or iPasts having APOE3/3 genotype and mouse primary neurons were co-cultured, and morphological changes thereof were investigated.

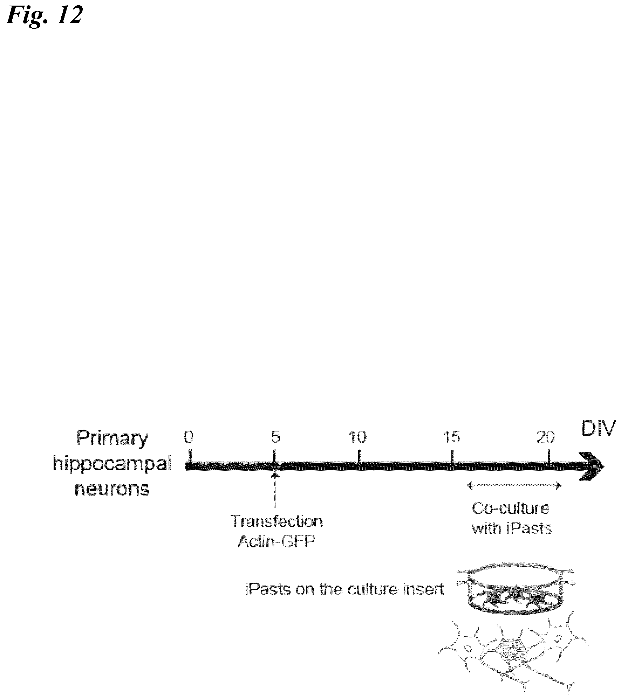

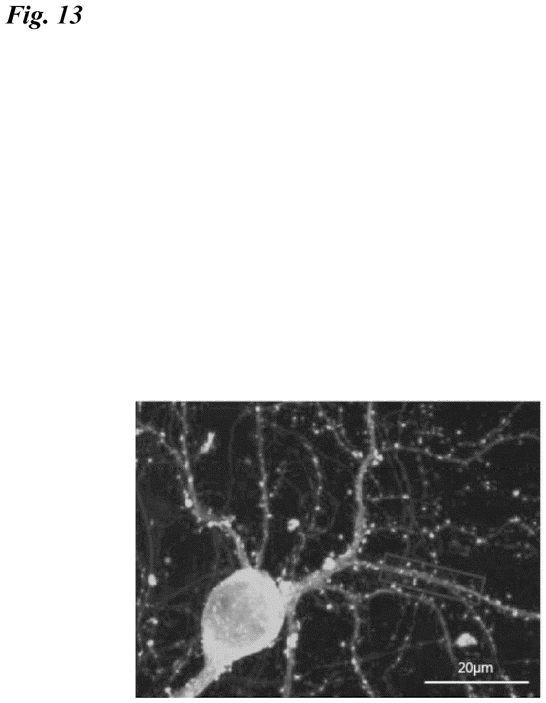

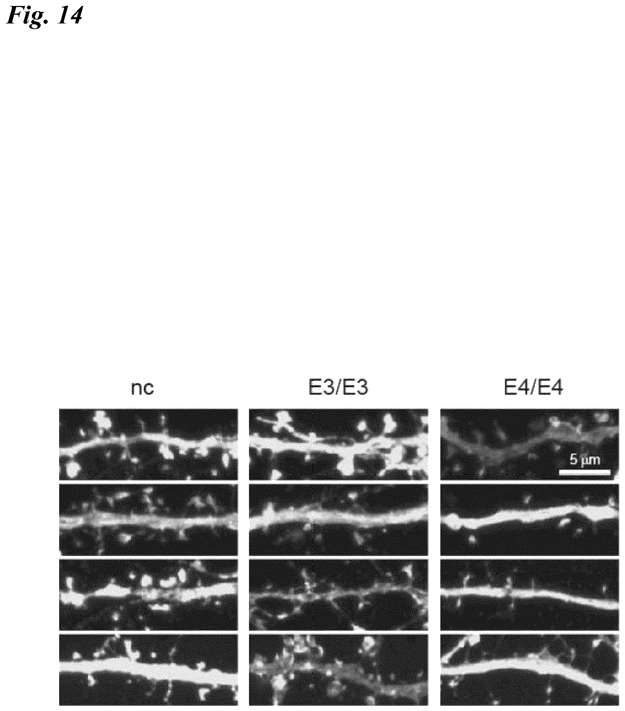

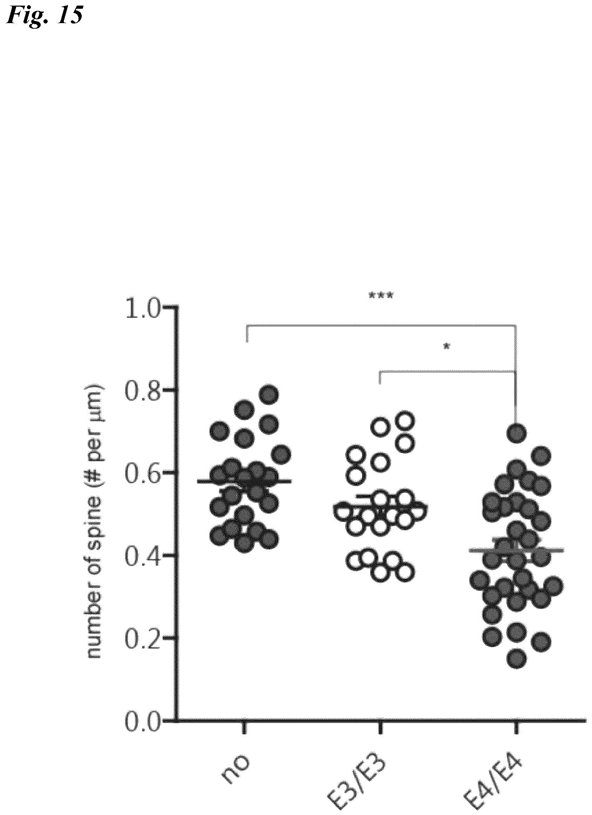

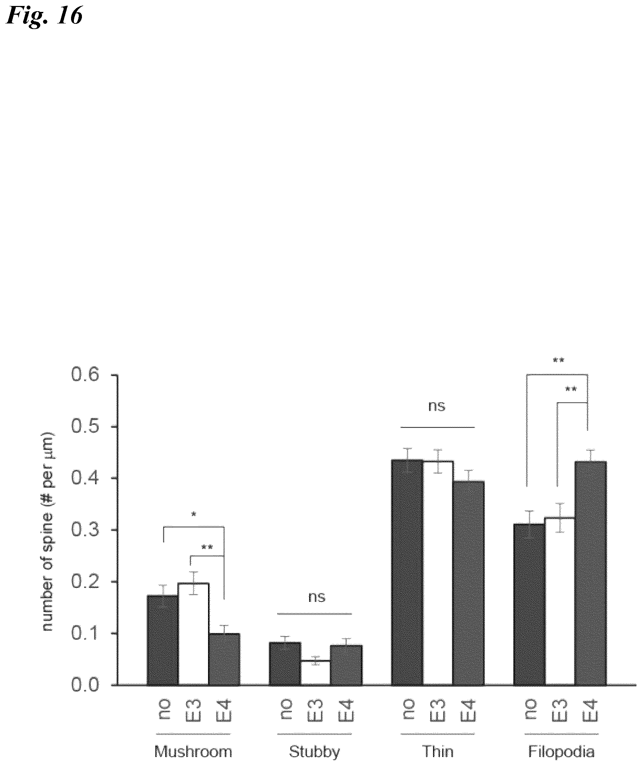

FIG. 12 is a diagram showing an outline of co-culture of iPasts having APOE4/4 genotype or iPasts having APOE3/3 genotype with mouse primary neurons. In the co-culture system, the iPasts were cultured in the culture insert. As in the representative fluorescence images shown in FIG. 13, clear fluorescence observation of dendritic spines was possible in the mouse primary neurons transfected with Actin-GFP. FIG. 14 shows fluorescence images of dendrites of mouse primary neurons co-cultured with the iPasts having APOE4/4 genotype or the iPasts having APOE3/3 genotype, and FIG. 15 shows the number of dendritic spines observed per µm of dendrite. In FIG. 15, * means that the p value in Tukey's test is less than 0.05, and *** means that the p value in Tukey's test is less than 0.001. Interestingly, the dendritic spine density was significantly reduced in the primary neurons co-cultured with the iPasts having APOE4/4 genotype. When viewed in the shape of the dendritic spines, as shown in FIG. 16, mature Mushroom-type spines were significantly decreased, whereas immature Filopodia-type spines were significantly increased. These results showed that the morphological change observed in this Example was large as compared with the difference in amount of secreted APOE in Example 2, suggesting that the decrease in dendritic spines of primary neurons in the presence of the iPasts having APOE4/4 genotype is caused by another secretion other than APOE protein.

### <Example 4: Search for mRNA Whose Expression Level Varies in iPast Having APOE4/4 Genotype>

In order to elucidate a molecular mechanism in which a decrease in dendritic spines of primary neurons occurs in the presence of iPast having APOE4/4 genotype, genes having different expression levels between the iPast having APOE4/4 genotype and the iPasts having APOE3/3 genotype were searched by microarray analysis (transcriptome analysis) using total RNA. The analysis was performed according to the method described in "Transcriptome Analysis" in the Experimental Method section.

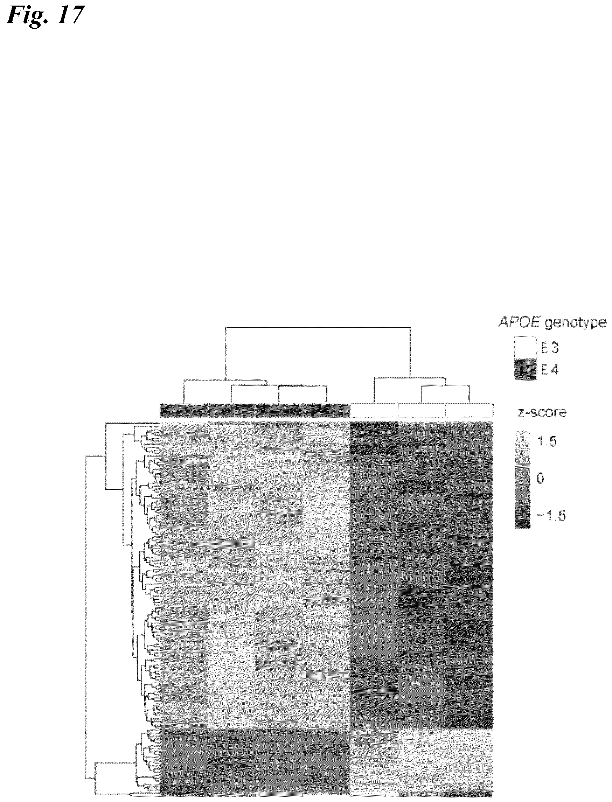

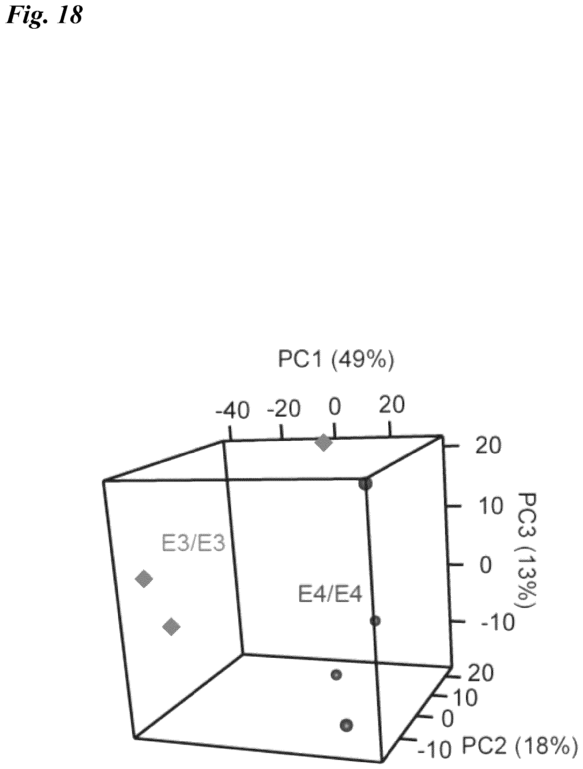

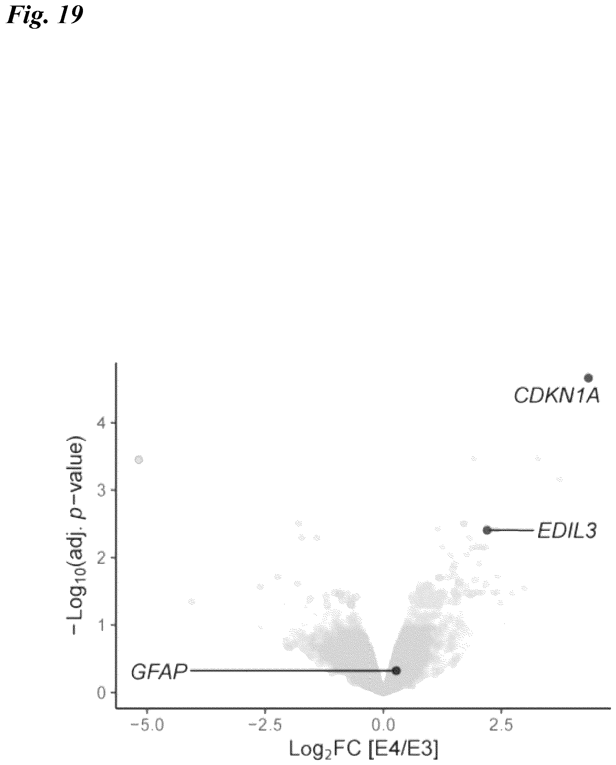

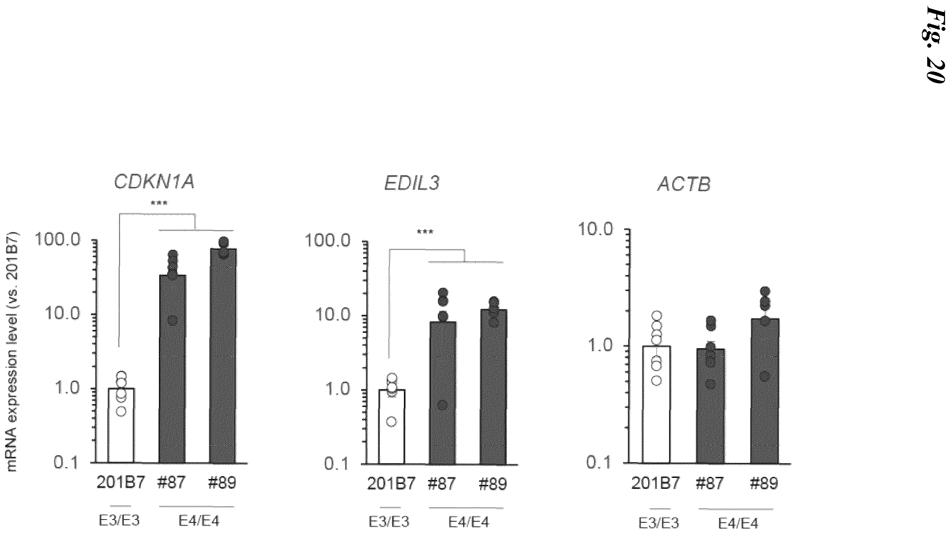

FIG. 17 shows results of clustering analysis as a heat map for the z-score of logFC (fold change) of the iPasts having APOE4/4 genotype relative to the iPasts having APOE3/3 genotype (score obtained by normalizing logFC so that the average value is 0 and that the standard deviation is 1). FIG. 18 shows results of principal component analysis (PCA) on the transcriptome of the iPasts having APOE3/3 genotype and the iPasts having APOE4/4 genotype. FIG. 19 is a two-dimensional plot of the results in FIG. 17. These results revealed that the iPasts having APOE3/3 genotype and the iPasts having APOE4/4 genotype have similar but different transcriptomes. As shown in FIG. 20, among the found mRNAs, CDKN1A and EDIL3 whose expression levels were increased in the iPasts having APOE4/4 genotype, and β actin (ACTB), an index of the number of cells, were also quantified by quantitative reverse transcription PCR (qRT-PCR), and it was confirmed that the expression levels thereof were increased in the iPasts having APOE4/4 genotype in comparison with those in the iPasts having APOE3/3 genotype.

### <Example 5: Study on Expression Level of Immune-related Gene>

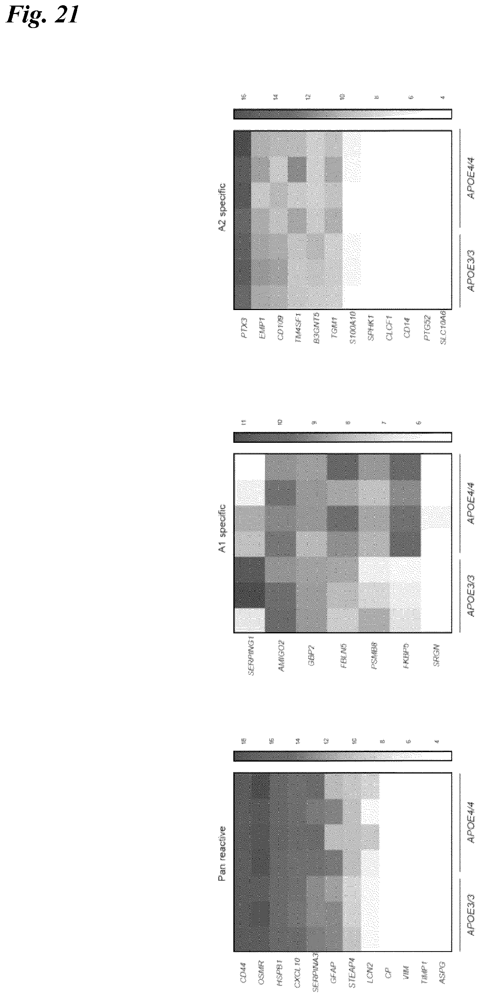

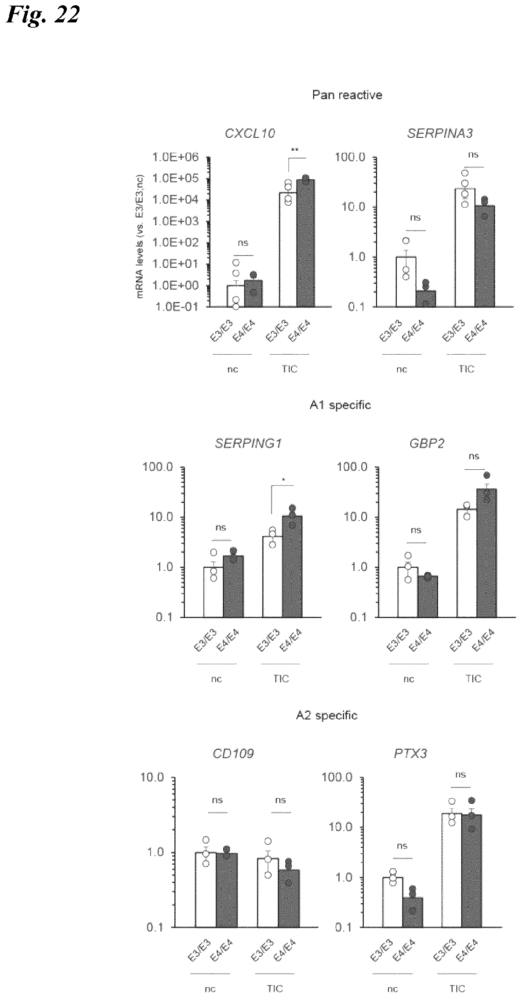

Among the mRNAs, found in Example 4, whose expression levels varied between the iPasts having APOE3/3 genotype and the iPasts having APOE4/4 genotype, the expression levels of immune-related genes known to be associated with the progression of Alzheimer's disease were examined in detail. FIGS. 21 and 22 show results of measuring the expression levels of genes known to be specifically expressed in Pan-Reactive, A1-like reactive (A1 specific), or A2-like reactive (A2 specific) astrocytes by the same method as in Example 4. According to these results, in the markers of the Pan-Reactive and A2-like reactive astrocytes, no significant difference was found between the iPasts having APOE3/3 genotype and the iPasts having APOE4/4 genotype, while in the marker of A1-like reactive astrocytes, an increase in expression level was found in the iPasts having APOE4/4 genotype. However, the increase amount was smaller than the degree of the neuronal morphological change found in Example 3, suggesting that genes other than the immune-related genes examined in Example 4 are related to the neuronal morphological change by co-culture with the iPasts having APOE4/4 genotype.

### <EXAMPLE 6: Search for Secretion of iPast Having APOE4/4 Genotype Causing Neuronal Morphological Changes>

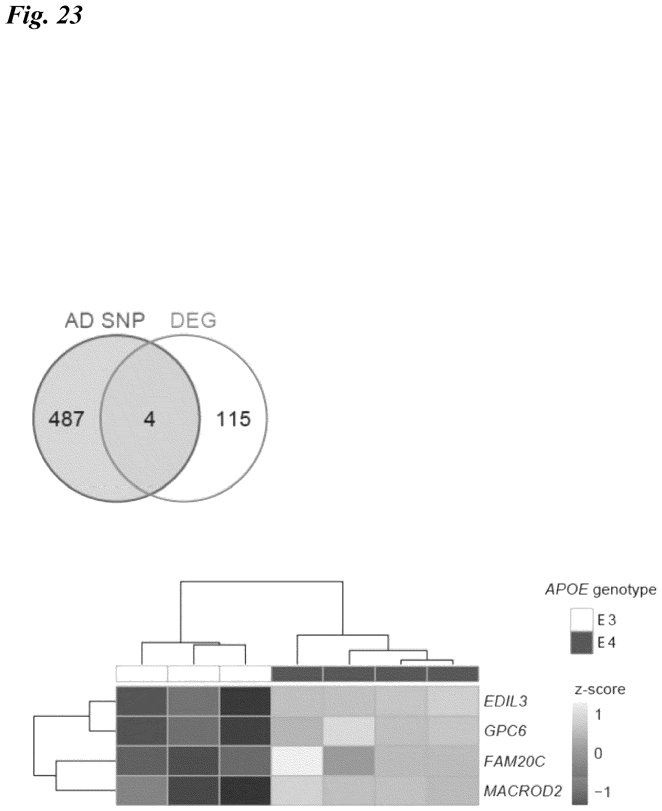

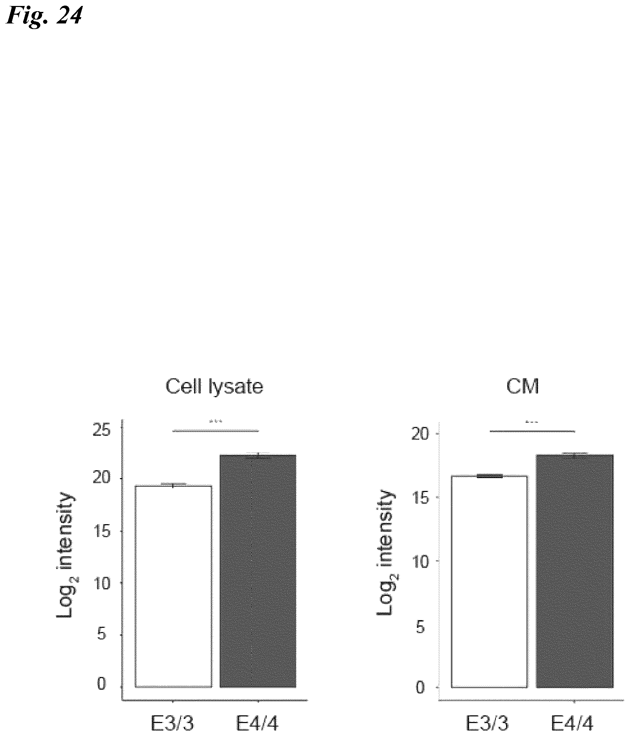

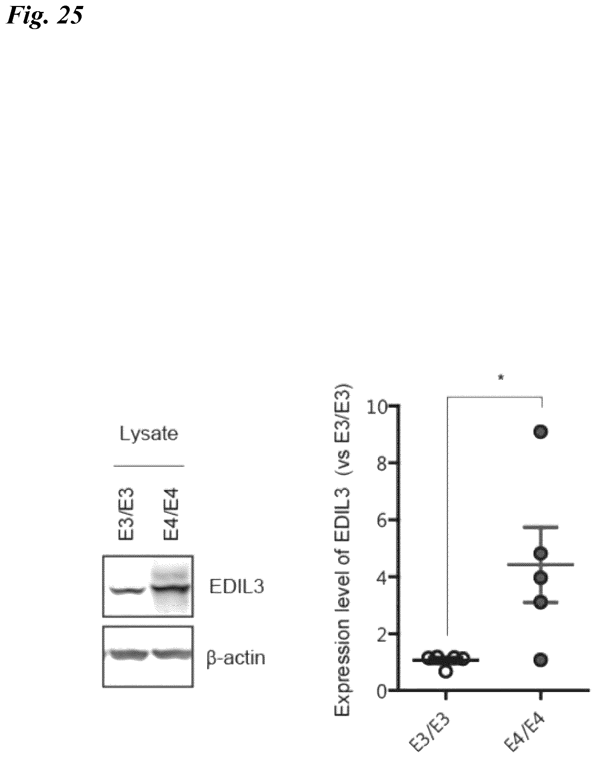

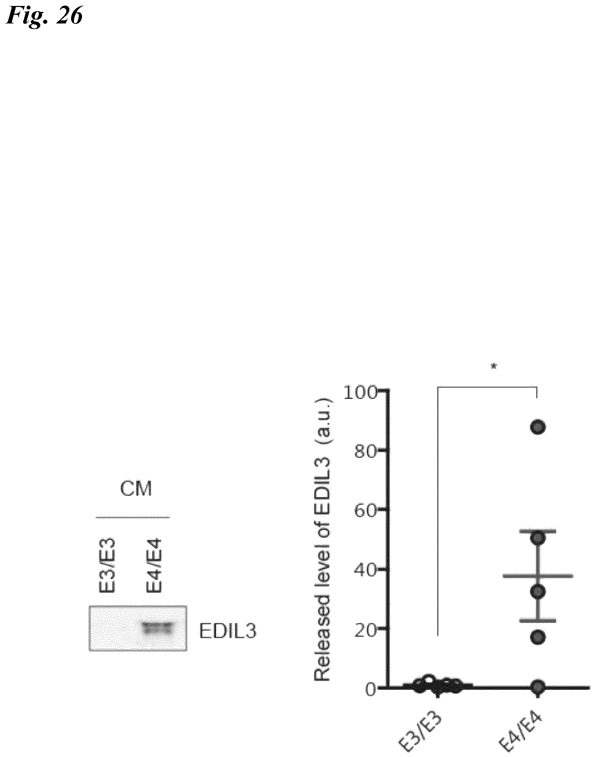

A secretion of the iPasts having APOE4/4 genotype was studied, which causes morphological changes in the neurons generated by co-culture with the iPasts having APOE4/4 genotype. First, as shown in FIG. 23, four genes: EDIL3 (EGF Like Repeats And Discoidin Domains 3), GPC6 (Glypican 6), FAM20C (Family with sequence similarity 20 member C), and MACROD2 (Mono-ADP Ribosylhydrolase 2) were found as genes common to 119 mRNAs, found in Example 4, in which the RNA amount was increased in the iPasts having APOE4/4 genotype as compared with that in the iPasts having APOE3/3 genotype, and 491 genes (AD GWAS genes, AD SNP) found in the genome-wide association analysis in Non Patent Literature 12 as genes whose single nucleotide polymorphism may be associated with Alzheimer's disease. Subsequently, regarding EDIL3 among these, the amount of EDIL3 protein in a an iPast lysate and a culture medium (CM) in which the iPasts were cultured was evaluated by mass spectrometry analysis. As shown in FIG. 24, the expression level and secretion level of EDIL3 in the iPasts having APOE4/4 genotype were significantly higher than those in the iPasts having APOE3/3 genotype. Furthermore, as a result of lysating iPast and evaluating the expression level of EDIL3 protein by Western blotting, as shown in FIG. 25, the expression level of EDIL3 in the iPasts having APOE4/4 genotype was significantly higher than that in the iPasts having APOE3/3 genotype. Furthermore, the amount of EDIL3 secreted into the culture medium in which iPast was cultured was similarly evaluated by Western blotting. As a result, as shown in FIG. 26, the amount of EDIL3 secreted by the iPasts having APOE4/4 genotype was significantly higher than that of the iPasts having APOE3/3 genotype. In FIGS. 24 to 26, the data are shown as the mean value ± standard error of the mean (Mean ± S.E., n = 3 (FIG. 24), n = 4 to 5 (FIGS. 24 and 25)), * and *** mean that p values in Student's t test are less than 0.05 and less than 0.001, respectively. From the above results, EDIL3 was found as a candidate for the secretion of the iPasts having APOE4/4 genotype, which causes morphological changes in the neurons generated by co-culture with the iPasts having APOE4/4 genotype.

### <Example 7: Examination of Influence of EDIL3 Secreted by Cell on Mouse Primary Neuron Using Cell Overexpressing EDIL3>

In order to examine the influence of EDIL3 found in Example 6 on mouse primary neurons, EDIL3 was overexpressed in a cultured cell line, and the influence of EDIL3 secreted extracellularly by the cell on the morphology of mouse primary neurons was examined.

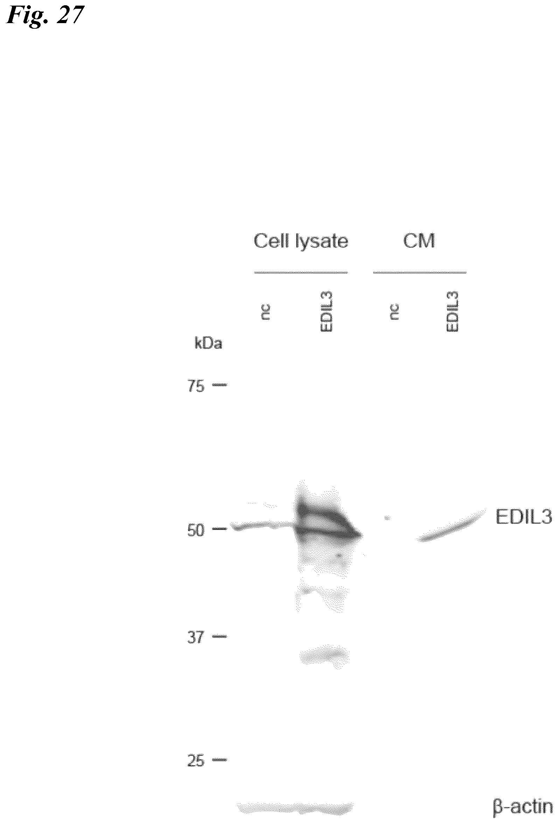

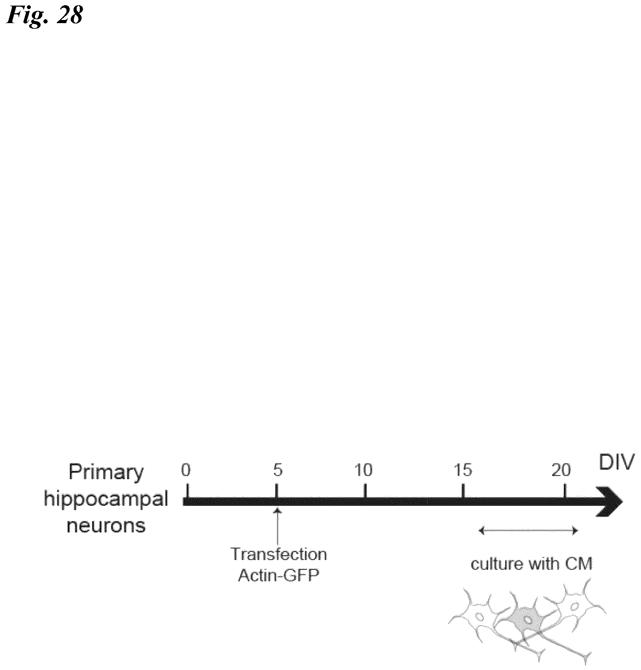

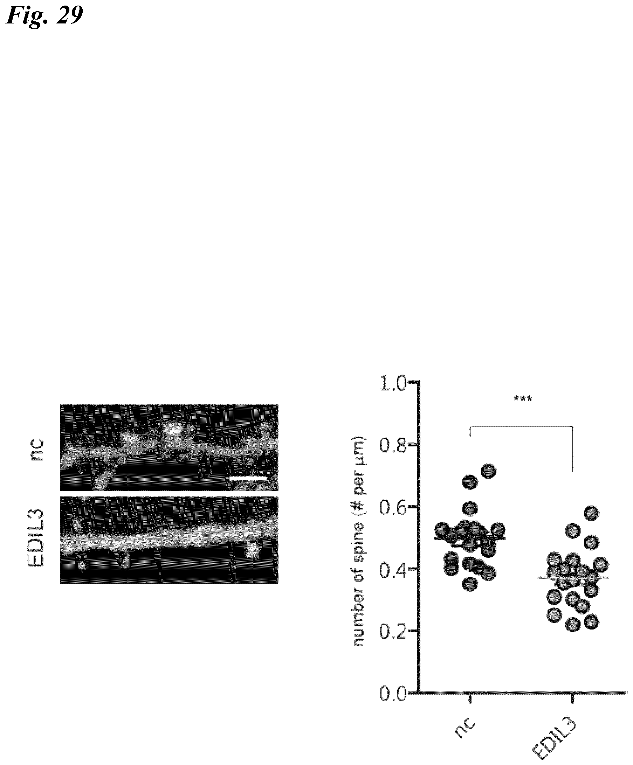

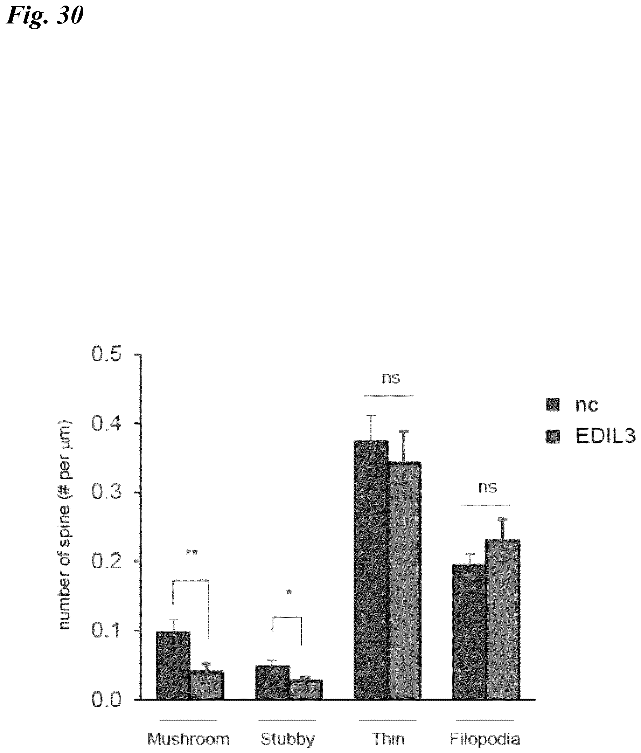

EDIL3 was transduced into HEK293 cells by a conventional method using the plasmid for expressing EDIL3 constructed according to the method described in "Construction of Expression Plasmid" in the Experimental method section. As a control (nc), HEK293 cells transduced with DsRed, a fluorescent protein instead of EDIL3, were used. According to Western blotting performed in the same manner as in Example 6, EDIL3 was transduced into the cells, and EDIL3 was secreted extracellularly from the cells, as shown in FIG. 27. Therefore, the culture medium in which the HEK293 cells transduced with EDIL3 were cultured was recovered, and, as shown in FIG. 28, mouse primary neurons were cultured in the culture medium during the period of Days 16 to 21 of culture (DIV 16 to 21), and the influence of exposure to EDIL3 on the mouse primary neurons was evaluated. FIG. 29 shows fluorescence images of Actin-GFP (left side) and results of quantifying the number of dendritic spines per µm (right side), in the dendrites of the mouse primary neurons after culture. In FIG. 29, *** means that the p value in Student's t-test is less than 0.001. According to FIG. 29, the number of dendritic spines was significantly reduced in the mouse primary neurons exposed to EDIL3 in culture medium compared to the control. In addition, also for each dendritic spine shape, as shown in FIG. 30, the Mushroom-type spines were significantly reduced, similarly to the tendency of the results of Example 3.

### <Example 8: Study on Influence of EDIL3 Secreted by Cell on Mouse Primary Neuron by Comparison in Presence or Absence of EDIL3 Knockdown in Cocultured iPast>

To examine the influence of EDIL3 found in Example 6 on the mouse primary neurons, the morphology of the mouse primary neurons co-cultured with EDIL3 knocked-down iPasts having APOE4/4 genotype was evaluated.

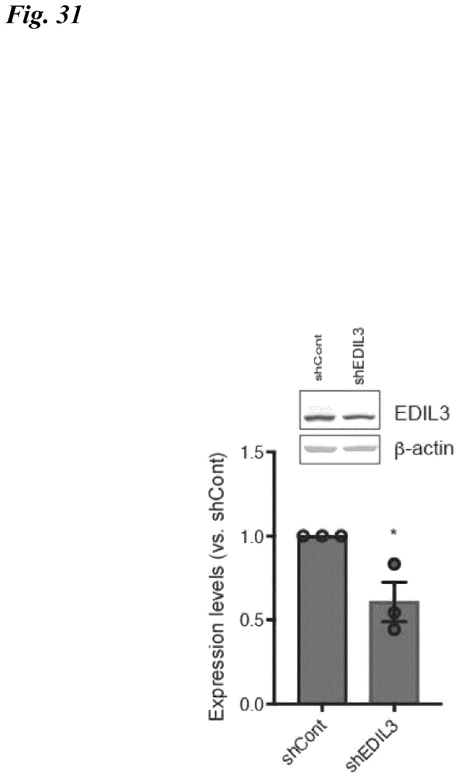

shEDIL3 shown in SEQ ID NO: 29 was introduced into the iPasts having APOE4/4 genotype using a lentivirus to knock down EDIL3. As a control, instead of shEDIL3, iPasts having APOE4/4 genotype into which shcontrol shown in SEQ ID NO: 30 was introduced were prepared. The results of evaluating the expression level of EDIL3 protein in these iPasts having APOE4/4 genotype by Western blotting are as shown in FIG. 31. In the iPasts into which shEDIL3 was introduced, the expression of EDIL3 was significantly suppressed. Similarly, for the iPasts having APOE3/3 genotype, iPasts into which shEDIL3 or shcontrol was introduced were prepared.
shEDIL3:5'-GTGGAATATGTACAGATCT-3' (SEQ ID NO: 29)
shcontrol:5'-TAAGGTTAAGTCGCCCTCG-3' (SEQ ID NO: 30)

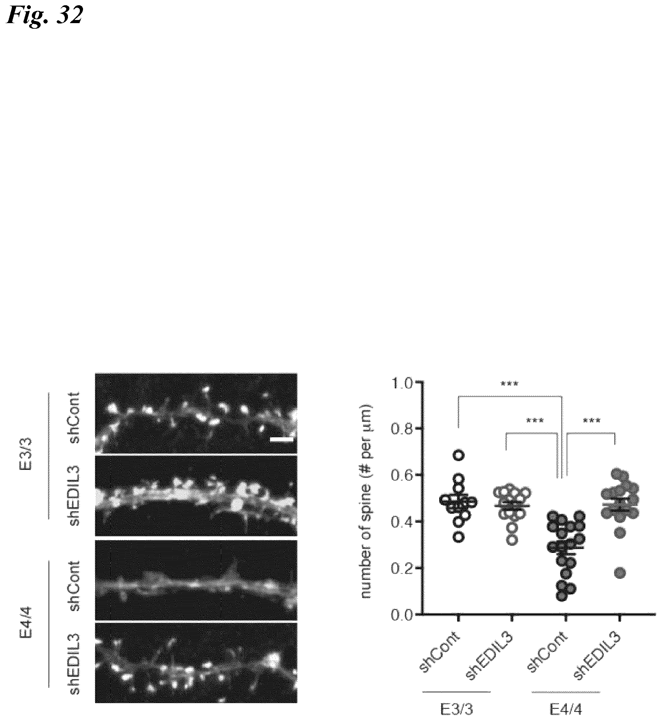

The thus-prepared iPasts having APOE4/4 genotype or iPasts having APOE3/3 genotype into which shEDIL3 or shcontrol was introduced was co-cultured with mouse primary neurons according to the same protocol as in Example 7, and the morphology of the mouse primary neurons was evaluated. FIG. 32 shows fluorescence images of Actin-GFP (left side) and results of quantifying the number of dendritic spines per µm (right side), in the dendrites of the mouse primary neurons after culture. The results in FIG. 32 are shown as the mean value ± standard error of the mean (Mean ± S.E., n = 17 neurons from 3 independent culture batches), where *** means that the p-value in Tukey's test is less than 0.001. According to FIG. 32, the number of dendritic spines was significantly reduced in the mouse primary neurons co-cultured with the iPasts having APOE4/4 genotype into which shcontrol was introduced (that is, EDIL3 was not knocked down), whereas in the mouse primary neurons co-cultured with the iPasts having APOE4/4 genotype into which shEDIL3 was introduced (that is, EDIL3 was knocked down), the number of dendritic spines was comparable to that in the mouse primary neurons co-cultured with the iPasts having APOE3/3 genotype, and the reduction in the number was significantly suppressed. The results of Examples 7 and 8 showed that EDIL3 has the action of decreasing dendritic spines in primary neurons, that is, the secretion of the iPasts having APOE4/4 genotype, which has the action of decreasing dendritic spines in primary neurons, is EDIL3.

### <Example 9: Evaluation of Amount of EDIL3 in Amyloid Plaque in Brain of Alzheimer's Disease Patient Having APOE4 Genotype>

Based on the above results, whether accumulation of EDIL3 protein was observed in amyloid plaques in the postmortem brain of Alzheimer's disease patients having APOE4 genotype was examined.

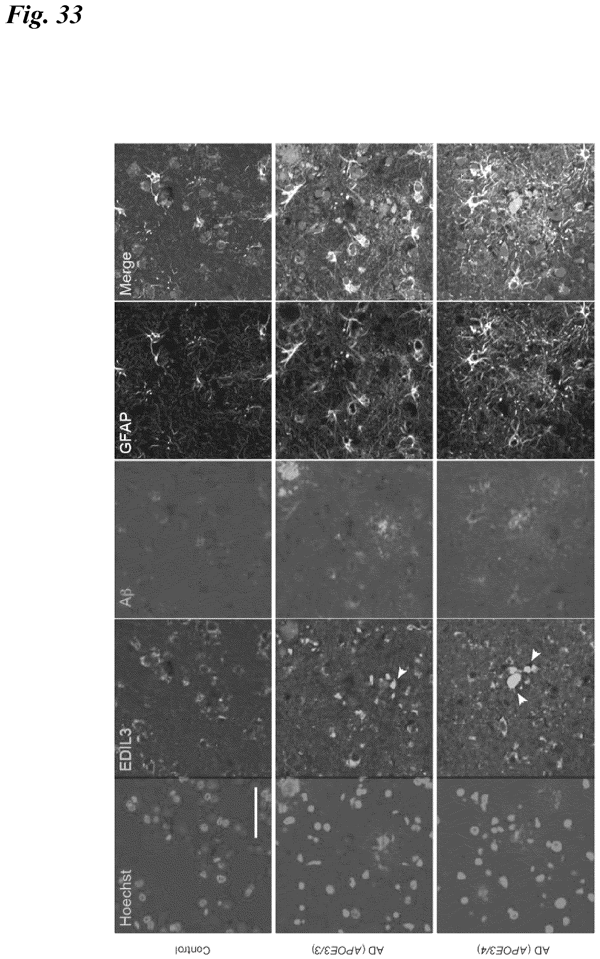

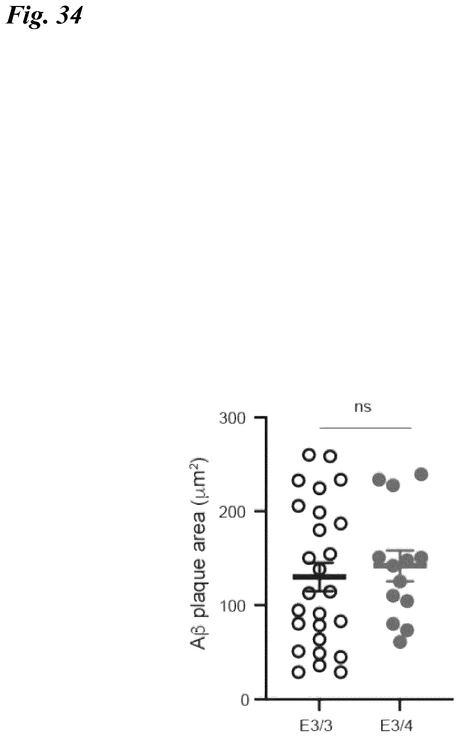

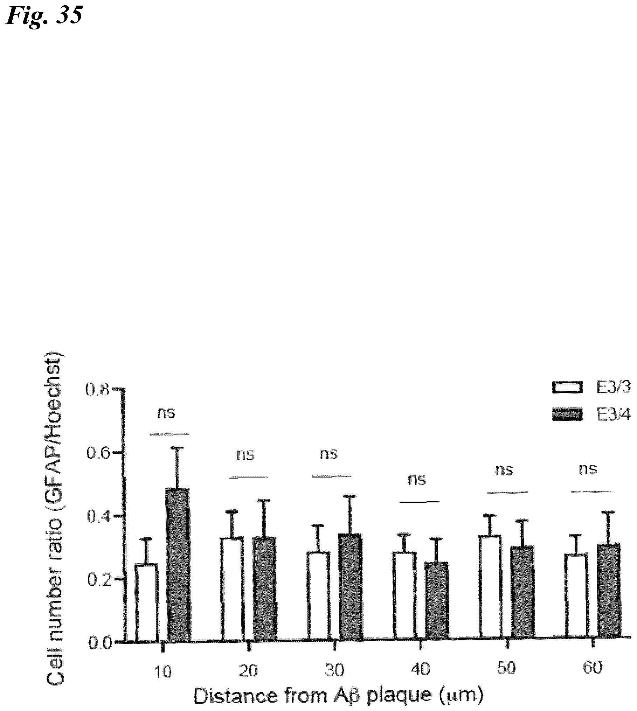

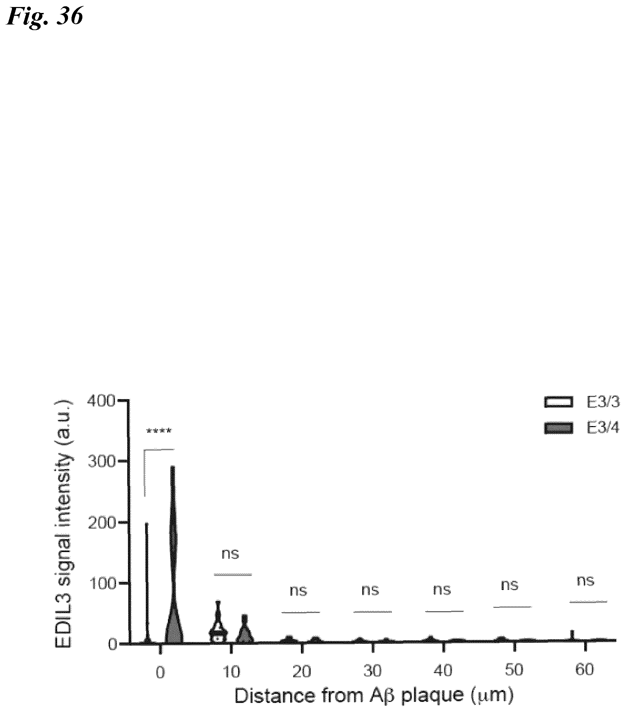

EDIL3, amyloid β (Aβ), and GFAP were immunofluorescently stained and cell nuclei were stained with Hoechst, in samples collected from the parietal lobe section of the postmortem brain of Alzheimer's disease patients (two patients: APOE3/3 genotype, two patients: APOE3/4 genotype) or humans (controls) who did not show a symptom of Alzheimer's disease. The fluorescence images of the stained samples are shown in FIG. 33. According to FIG. 33, accumulation of EDIL3 indicated by arrows was observed in amyloid plaques in the brain of Alzheimer's disease patients, whereas accumulation of EDIL3 was not observed in amyloid plaques in the brain of control patients. FIG. 34 shows the area of amyloid plaques in the brain of Alzheimer's disease patients (n = 13 to 26). The proportion of GFAP-positive cells (the number of cells in which a fluorescence image of GFAP was observed/the number of Hoechst stained cells) according to the distance from amyloid plaques in the brain of the Alzheimer's disease patient is shown in FIG. 35. FIG. 36 shows the fluorescence intensity derived from a labeled anti-EDIL3 antibody according to the distance from amyloid plaques in the brain of the Alzheimer's disease patients. The data in FIGS. 35 and 36 are shown as results for 13 to 26 amyloid plaques, and ns and **** mean that the p values in Sidak's multiple comparisons test following a two-way ANOVA are 0.05 or more and less than 0.0001, respectively. According to FIG. 34, no significant difference in size of amyloid plaques was found in whether or not Alzheimer's disease patients had APOE4 genotype. In addition, according to FIG. 35, since no significant difference in proportion of GFAP-positive cells was found in whether or not Alzheimer's disease patients had APOE4 genotype, revealing that an increase in astrocytes did not occur in the brain of the patients having APOE4 genotype. In addition, according to FIG. 36, in the brain of the patients having APOE4 genotype, the fluorescence intensity was remarkably high in regions overlapping amyloid plaques (ring with a distance of 0 µm) in the concentric rings for the amyloid plaques, and remarkable accumulation of EDIL3 was observed in the vicinity of amyloid plaques, as compared with that in the brain of the patients not having APOE4 genotype. This suggested that EDIL3 is involved in Alzheimer's disease in humans, and is deeply involved in Alzheimer's disease in patients having APOE4 genotype, in particular. In addition, this suggests that in the brain of Alzheimer's disease patients (particularly, in the brain of Alzheimer's disease patients having APOE4 genotype), EDIL3 is excessively deposited together with amyloid β peptide, and causes synaptic impairment in the vicinity of the plaques.

## Claims

1. A kit for diagnosing or assisting diagnosis of Alzheimer's disease, the kit comprising:
a nucleic acid primer or nucleic acid probe configured to detect an mRNA encoding EDIL3;
a nucleic acid primer or nucleic acid probe configured to detect a cDNA complementary to the mRNA; or
an anti-EDIL3 antibody or an EDIL3 binding fragment thereof.

2. The kit according to claim 1, wherein a subject to be diagnosed is a subject having APOE4 genotype.

3. The kit according to claim 1 or 2, comprising an instruction for use.

4. A method for diagnosing Alzheimer's disease, the method comprising quantifying an mRNA encoding EDIL3, a cDNA complementary to the mRNA, or EDIL3 in a subject.

5. A method for assisting diagnosis of Alzheimer's disease, the method comprising quantifying an mRNA encoding EDIL3, a cDNA complementary to the mRNA, or EDIL3 in a subject.

6. The method according to claim 4 or 5, wherein a higher quantified amount of EDIL3 compared to a control indicates that the subject has or is likely to have Alzheimer's disease.

7. The method according to any one of claims 4 to 6, comprising detecting the presence or absence of APOE4 genotype of the subject.

8. A pharmaceutical composition for treating Alzheimer's disease, comprising a substance that inhibits EDIL3.

9. The pharmaceutical composition according to claim 8, wherein the substance that inhibits EDIL3 is a substance that suppresses expression of EDIL3, a substance that promotes degradation of EDIL3, or a substance that inhibits the function of EDIL3.

10. The pharmaceutical composition according to claim 8 or 9, wherein the substance that inhibits EDIL3 is a nucleic acid that suppresses expression of EDIL3, or an anti-EDIL3 antibody or an EDIL3 binding fragment thereof.

11. The pharmaceutical composition according to any one of claims 8 to 10, which is a pharmaceutical composition for treating a human subject having APOE4 genotype.

12. A method for screening for a substance that inhibits EDIL3, the method comprising:
culturing a neuron in a medium containing EDIL3 and a test substance; and
detecting a phenotype of Alzheimer's disease from the neuron.

13. A method for screening for a substance that inhibits EDIL3, the method comprising:
introducing a nucleic acid for expressing EDIL3 into a cell or microorganism;
culturing the cell or microorganism;
recovering a culture supernatant obtained by culturing the cell or microorganism;
culturing a neuron in a medium containing the culture supernatant and a test substance; and
detecting a phenotype of Alzheimer's disease from the neuron.

14. The screening method according to claim 12 or 13, wherein the neuron is a neuron having APOE4 genotype.

15. The screening method according to any one of claims 12 to 14, wherein the detecting a phenotype of Alzheimer's disease from the neuron comprises detecting a change in number of spines of the neuron.

16. The screening method according to any one of claims 12 to 15, wherein the substance that inhibits EDIL3 is a substance for treating Alzheimer's disease.

17. A method for screening for a substance that inhibits EDIL3, for treating Alzheimer's disease, the method comprising:
culturing a cell expressing EDIL3 in a medium containing a test substance; and
evaluating expression or secretion of EDIL3.

18. The screening method according to claim 17, wherein the evaluating expression or secretion of EDIL3 is quantifying an mRNA encoding EDIL3, a cDNA complementary to the mRNA, or EDIL3.

19. The screening method according to claim 17 or 18, wherein the quantification of EDIL3 is performed using an anti-EDIL3 antibody or an EDIL3 binding fragment thereof.

20. The screening method according to any one of claims 12 to 19, wherein the substance that inhibits EDIL3 is a substance that suppresses expression of EDIL3, a substance that promotes degradation of EDIL3, or a substance that inhibits the function of EDIL3.
